# EUROPEAN PATENT APPLICATION

(11) **EP 1 314 721 A1**
(43) Date of publication of application: **28.05.2003**
(21) Application number: 01958495.2
(22) Date of filing: 27.08.2001
(51) Int. Cl.: C07C 259/06, C07C 275/42, C07C 311/21, C07C 311/29, C07C 311/44, C07C 311/46, C07C 335/22, C07D 295/22, C07D 215/36, C07D 213/74, C07D 213/89, A61K 31/18, A61K 31/17, A61K 31/4453, A61K 31/47, A61K 31/4406, A61K 31/4409, A61K 31/4402, A61K 31/44

(54) **NOVEL PROPENOHYDROXAMIC ACID DERIVATIVES**

(30) Priority: 31.08.2000 JP 2000263094
(71) Applicant: WAKUNAGA PHARMACEUTICAL CO., LTD., Osaka-shi, Osaka-fu 532 (JP)
(72) Inventor: HIRATA, Terukage, WAKUNAGA PHARMACEUTICAL CO. LTD., TAKATA-GUN, Hiroshima 739-1195 (JP); MISUMI, Keiji, WAKUNAGA PHARMACEUTICAL CO. LTD., TAKATA-GUN, Hiroshima 739-1195 (JP); ITO, Kenji, C/O WAKUNAGA PHARMACEUTICAL CO. LTD., TAKATA-GUN, Hiroshima 739-1195 (JP); INOKUMA, Kenichi, WAKUNAGA PHARMACEUTICAL CO. Ltd., TAKATA-GUN, Hiroshima 739-1195 (JP); KATAYAMA, Kimiko, WAKUNAGA PHARMACEUTICAL CO. LTD., TAKATA-GUN, Hiroshima 739-1195 (JP)
(74) Representative: Hartz, Nikolai F., Dr.
(86) International application number: JP0107292
(87) International publication number: WO02018326

(57) **Abstract**

Provided are a propenohydroxamic acid derivative represented by the following formula (1): 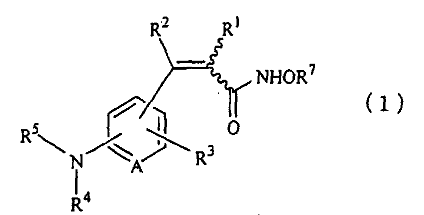 [wherein, R¹ represents a hydrogen atom, an alkyl group or a halogen atom, R² represents a cycloalkyl group, a substituted or unsubstituted aryl group or a substituted or unsubstituted heteroaryl group, R³ represents a hydrogen atom or a halogen atom, R⁴ represents a hydrogen atom, a substituted or unsubstituted alkyl group or a substituted or unsubstituted alkenyl group, R⁵ represents R⁶CO-, R⁶SO₂-, R⁶NRCO- or R⁶NHCS- (in which, R⁶ represents a substituted or unsubstituted alkyl or cycloalkyl group, a cyclic amino group, a substituted or unsubstituted aryl group or a substituted or unsubstituted heteroaryl group), R⁷ represents a hydrogen atom or a protecting group and A represents CH, a nitrogen atom or an oxidized nitrogen atom], or salt thereof; and a medicament containing the propenohydroxamic acid derivative or salt thereof.

The compound (1) or salt thereof has excellent TACE inhibitory activity and is therefore useful as a medicament for preventing and/or treating diseases such as septicemia, rheumatoid arthritis, osteoarthritis, infectious diseases, autoimmune diseases, malignant neoplasm, collagenosis, chronic ulcerative colitis, MOF and insulin-independent diabetes.

## Description

### Technical Field

The present invention relates to novel propenohydroxamic acid derivatives or salts thereof, and medicaments containing the same.

### Background Art

Tumor necrosis factor-α (TNF-α) is one of cytokines produced by activated B cells, T cells, macrophages, NK cells or the like cells. It is known that TNF-α has, as well as strong antitumor activity, a variety of physiological activities not only for tumor cells but also for normal cells and plays an important role as various inflammatory mediators. Excessive extracellular release of TNF-α is presumed to cause diseases such as septicemia, rheumatoid arthritis, osteoarthritis and chronic ulcerative colitis. TNF-α produced by fat cells is known to have a close relation with a cause and morbid conditions of diabetes. In non-insulin-dependent diabetes mellitus (NIDDM), its role as a mediator of insulin resistance linked to obesity is attracting attentions. Moreover, its intimate involvement in diseases with organ disorders such as multiple organ failure (MOF) is also known.

It has recently been revealed that an enzyme (TNF-α converting enzyme; TACE) causing release of TNF-α is metalloproteinase. Based on the concept that the above-described diseases can be prevented or treated by controlling or inhibiting the action of TNF-α through a TACE action inhibitor, development of a TACE inhibitor has been carried out.

Compounds, for example, as described in Journal of Leukocyte Biology, **57**, 774(1995), Nature, **370**, 218(1994), and Nature, **370**, 558(1994) are known to have TACE inhibitory activity. These compounds have inhibitory action on extracellular matrix metalloproteinases (MMP). Since they also act on a plurality of MMPs other than TACE, there is a potential danger that they may exhibit undesirable action.

Substances selectively inhibiting TACE have recently been reported (Japanese Patent Application No. Hei 7-507668, Japanese Patent Application No. Her 10-255899). Their activity is however not sufficient and medicaments suited for clinical applications have not yet been found.

An object of the present invention is therefore to provide a novel compound capable of selectively inhibiting TACE and useful as a preventive or remedy for various diseases resulting from excessive extracellular release of TNF-α, for example, septicemia, rheumatoid arthritis, osteoarthritis, infectious diseases, autoimmune diseases, malignant neoplasm, collagenosis, chronic ulcerative colitis, MOF and insulin-independent diabetes.

### Disclosure of the Invention

With the foregoing in view, the present inventors have searched for a substance having TACE inhibitory action. As a result, it has been found that novel propenohydroxamic acid derivatives (1) described below and salts thereof have excellent TACE inhibitory activity and are therefore useful as a medicament, leading to the completion of the present invention.

In one aspect of the present invention, there is thus provided a propenohydroxamic acid derivative represented by the following formula (1): 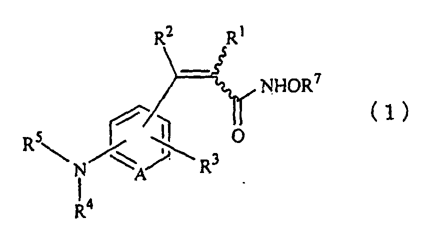 [wherein, R¹ represents a hydrogen atom, an alkyl group or a halogen atom, R² represents a cycloalkyl group, a substituted or unsubstituted aryl group or a substituted or unsubstituted heteroaryl group, R³ represents a hydrogen atom or a halogen atom, R⁴ represents a hydrogen atom, a substituted or unsubstituted alkyl group or a substituted or unsubstituted alkenyl group, R⁵ represents R⁶CO-, R⁶SO₂-, R⁶NHCO- or R⁶NHCS- (in which, R⁶ represents a substituted or unsubstituted alkyl or cycloalkyl group, a cyclic amino group, a substituted or unsubstituted aryl group or a substituted or unsubstituted heteroaryl group), R⁷ represents a hydrogen atom or a protecting group and A represents CH, a nitrogen atom or an oxidized nitrogen atom], or salt thereof.

In another aspect of the present invention, there is also provided a medicament containing the propenohydroxamic acid derivative or salt thereof.

In a further aspect of the present invention, there is also provided a TACE inhibitor containing the propenohydroxamic acid derivative or salt thereof.

In a still further aspect of the present invention, there is also provided a pharmaceutical composition containing the propenohydroxamic acid derivative or salt thereof and a pharmaceutically acceptable carrier.

In a still further aspect of the present invention, there is also provided use of the propenohydroxamic acid derivative or salt thereof for the preparation of a medicament.

In a still further aspect of the present invention, there is also provided a method of treating a disease selected from septicemia, rheumatoid arthritis, osteoarthritis, infectious diseases, autoimmune diseases, malignant neoplasm, collagenosis, chronic ulcerative colitis, MOF and insulin-independent diabetes.

### Best Mode for Carrying Out the Invention

Each of the substituents in the formula (1) which represents the compound of the present invention will hereinafter be described.

R¹ represents a hydrogen atom, an alkyl group or a halogen atom. As this alkyl group, linear or branched C₁₋₈ alkyl groups can be mentioned as examples, of which linear or branched C₁₋₅ alkyl groups are preferred, with methyl, ethyl, n-propyl, isopropyl, n-butyl, isobutyl, tert-butyl and n-pentyl groups being especially preferred.

As the halogen atom represented by R¹, fluorine atom, chlorine atom, bromine atom and the like can be mentioned as examples, with fluorine and chlorine atoms being especially preferred.

Of these groups as R¹, preferred are a hydrogen atom and linear C₁₋₅ alkyl groups (especially, methyl, ethyl and n-propyl groups), with hydrogen atom being particularly preferred.

R² represents a cycloalkyl group, a substituted or unsubstituted aryl group or a substituted or unsubstituted heteroaryl group. As this cycloalkyl group, C₃₋₁₀ cycloalkyl groups can be mentioned as examples, of which C₃₋₈ cycloalkyl groups are preferred, with C₃₋₆ cycloalkyl groups such as cyclopropyl, cyclobutyl, cyclopentyl and cyclohexyl being especially preferred.

Examples of the aryl group in the substituted or unsubstituted aryl group represented by R² include aromatic C₆₋₁₄ hydrocarbon groups, of which phenyl and naphthyl groups are preferred.

Examples of the heteroaryl group in the substituted or unsubstituted heteroaryl group represented by R² include 5- to 14-membered monocyclic or bicyclic heteroaryl groups having 1 to 3 nitrogen atoms, oxygen atoms or sulfur atoms. If the heteroaryl group contains a nitrogen atom, the nitrogen atom may be an oxidized one. Examples of such heteroaryl group include pyridyl, pyridyl N-oxide, furanyl, thienyl, pyrrolyl, pyrimidinyl, imidazolyl, triazolyl, pyrazolyl, isothiazolyl, isoxazolyl, thiazolyl, oxazolyl, thiadiazolyl, pyridazinyl, pyrazinyl, benzofuryl, benzothienyl, benzopyranyl, quinolyl, phthalazinyl, naphthylizinyl, quinoxalinyl, quinazolinyl, cinnolinyl, indolyl and isoindolyl groups. Of these, 5- to 10-membered heteroaryl groups such as pyridyl, pyridyl N-oxide, furanyl, thienyl, thiazolyl, oxazolyl, naphthylizinyl and quinolyl are preferred.

The aryl or heteroaryl group represented by R² may have 1 to 3 substituents on the ring thereof. Examples of such a substituent include C₁₋₆ alkyl groups (such as methyl, ethyl, n-propyl, isopropyl, n-butyl, isobutyl, tert-butyl, n-pentyl and n-hexyl), mono-, di- or trihalogenoalkyl groups (such as trifluoromethyl and 2,2,2-trifluoroethyl), C₁₋₆ alkoxy groups (such as methoxy, ethoxy, propoxy, isopropoxy and butoxy), phenoxy group, halogen atoms (such as fluorine and chlorine), nitro group, hydroxy group, carboxy group, cyano group, sulfonyl group, sulfinyl group, sulfamoyl group, alkanoyl groups, aryloyl group and R⁹R¹⁰N-(in which, R⁹ represents a hydrogen atom or a lower alkyl group and R¹⁰ represents a hydrogen atom or groups similar to those exemplified as R⁵).

Examples of the lower alkyl group represented by R⁹ include methyl, ethyl, n-propyl, isopropyl, n-butyl, isobutyl, tert-butyl and n-pentyl groups. R¹⁰ represents a group similar to those exemplified as R⁵, more specifically, R⁶CO-, R⁶SO₂-, R⁶NHCO- or R⁶NHCS- (R⁶ representing a substituted or unsubstituted alkyl or cycloalkyl group, a cyclic amino group, a substituted or unsubstituted aryl group or a substituted or unsubstituted heteroaryl group). Specific examples are similar to those exemplified later as R⁵. Preferred is R⁶SO₂- (R⁶ representing a substituted or unsubstituted aryl group or a substituted or unsubstituted heteroaryl group) and as R⁶, a phenyl group substituted with a methoxy, nitro or the like group is preferred.

As R², a substituted or unsubstituted aryl group or a substituted or unsubstituted heteroaryl group is preferred.

R³ represents a hydrogen atom or a halogen atom. This halogen atom is similar to that exemplified as R¹, with fluorine and chlorine atoms being especially preferred.

R⁴ represents a hydrogen atom, a substituted or unsubstituted alkyl group or a substituted or unsubstituted alkenyl group. As this substituted or unsubstituted alkyl group, the alkyl groups exemplified as R¹ and these alkyl groups having 1 to 5 substituents can be mentioned. Examples of such a substituent include halogen atoms (such as chlorine and fluorine), nitro group, hydroxy group, carboxy group, cyano group, amino group, alkoxy groups (such as methoxy, ethoxy and propoxy), alkanoyl groups (such as formyl, acetyl, propionyl, butyryl, isobutyryl, valeryl, isovaleryl and pivaloyl), benzoyl group, aryl groups (such as phenyl and naphthyl) and heteroaryl groups (such as pyridyl, thienyl and furanyl).

Examples of the alkenyl group in the substituted or unsubstituted alkenyl group as R⁴ include linear or branched C₂₋₁₂ alkenyl groups, of which linear C₁₋₈ alkenyl groups are preferred, with linear C₁₋₆ alkenyl groups such as vinyl, allyl, 1-propenyl, 2-butenyl, 3-butenyl, 2-pentenyl, 3-pentenyl, 4-pentenyl, 2-hexenyl, 3-hexenyl, 4-hexenyl and 5-hexenyl groups being especially preferred. Examples of the substituent for these alkenyl groups include those exemplified as the substituents for the above-described alkyl groups.

R⁵ represents R⁶CO-, R⁶SO₂-, R⁶NHCO- or R⁶NHCS- (R⁶ representing a substituted or unsubstituted alkyl or cycloalkyl group, a cyclic amino group, a substituted or unsubstituted aryl group or a substituted or unsubstituted heteroaryl group). Examples of the substituted or unsubstituted alkyl group represented by R⁶ include those exemplified as R⁴.

Examples of the cycloalkyl group as R⁶ include C₃₋₁₀ cycloalkyl groups illustrated as R², of which C₃₋₆ cycloalkyl groups such as cyclopropyl, cyclobutyl, cyclopentyl and cyclohexyl groups are especially preferred.

Examples of the cyclic amino group represented by R⁶ include 4- to 8-membered, saturated or unsaturated cyclic amino groups such as azetidinyl, pyrrolidinyl, piperidino, piperazino and tetrahydropyridyl groups, of which 4- to 8-membered saturated cyclic amino groups such as azetidinyl, pyrrolidinyl, piperidino and piperazino groups are especially preferred.

Examples of the substituted or unsubstituted aryl group or substituted or unsubstituted heteroaryl group as R⁶ include those exemplified as R². Especially preferred as the substituted or unsubstituted aryl group are, as well as phenyl and naphthyl groups, phenyl and naphthyl groups each mono- or di-substituted with a C₁₋₈ alkyl group (such as methyl, ethyl, propyl, isopropyl or butyl), halogen atom (such as fluorine, chlorine or bromine), C₁₋₆ alkoxy group (such as methoxy, ethoxy, propoxy, isopropoxy or butoxy), phenoxy group, nitro group, amino group, or trihalogenoalkyl group (such as trifluoromethyl or 2,2,2-trifluoroethyl). Specific examples include 2-methylphenyl, 3-methylphenyl, 4-methylphenyl, 2-methoxyphenyl, 3-methoxyphenyl, 4-methoxyphenyl, 2-trifluoromethylphenyl, 3-trifluoromethylphenyl, 4-trifluoromethylphenyl, 2-fluorophenyl, 3-fluorophenyl, 4-fluorophenyl, 2-chlorophenyl, 3-chlorophenyl, 4-chlorophenyl, 2-nitrophenyl, 3-nitrophenyl, 4-nitrophenyl, 3-phenoxyphenyl, 4-phenoxyphenyl, 2,3-dimethylphenyl, 2,4-dimethylphenyl, 2,5-dimethylphenyl, 2, 6-dimethylphenyl, 3,4-dimethylphenyl, 3,5-dimethylphenyl, 2,3-dimethoxyphenyl, 2,4-dimethoxyphenyl, 2,5-dimethoxyphenyl, 2,6-dimethoxyphenyl, 3,4-dimethoxyphenyl, 3,5-dimethoxyphenyl, 2,3-difluorophenyl, 2, 4-difluorophenyl, 2,5-difluorophenyl, 2,6-difluorophenyl, 3,4-difluorophenyl, 3,5-difluorophenyl, 2,3-dinitrophenyl, 2,4-dinitrophenyl, 2,5-dinitrophenyl, 2,6-dinitrophenyl, 3,4-dinitrophenyl, 3,5-dinitrophenyl, 2-fluoro-3-methylphenyl, 2-fluoro-4-methylphenyl, 2-fluoro-5-methylphenyl, 2-fluoro-6-methylphenyl, 3-fluoro-2-methylphenyl, 4-fluoro-2-methylphenyl, 5-fluoro-2-methylphenyl, 3-fluoro-4-methylphenyl, 3-fluoro-S-methylphenyl, 4-fluoro-3-methylphenyl, 2-methoxy-3-methylphenyl, 2-methoxy-4-methylphenyl, 2-methoxy-5-methylphenyl, 2-methoxy-6-methylphenyl, 3-methoxy-2-methylphenyl, 4-methoxy-2-methylphenyl, 5-methoxy-2-methylphenyl, 3-methoxy-4-methylphenyl, 3-methoxy-5-methylphenyl, 4-methoxy-3-methylphenyl, 2-methyl-3-nitrophenyl, 2-methyl-4-nitrophenyl, 2-methyl-5-nitrophenyl, 2-methyl-6-nitrophenyl, 3-methyl-2-nitrophenyl, 4-methyl-2-nitrophenyl, 5-methyl-2-nitrophenyl, 3-methyl-4-nitrophenyl, 3-methyl-5-nitrophenyl, 4-methyl-3-nitrophenyl, 2-chloro-3-fluorophenyl, 2-chloro-4-fluorophenyl, 2-chloro-5-fluorophenyl, 2-chloro-6-fluorophenyl, 3-chloro-2-fluorophenyl, 4-chloro-2-fluorophenyl, 5-chloro-2-fluorophenyl, 3-chloro-4-fluorophenyl, 3-chloro-5-fluorophenyl, 4-chloro-3-fluorophenyl, 2-fluoro-3-methoxyphenyl, 2-fluoro-4-methoxyphenyl, 2-fluoro-5-methoxyphenyl, 2-fluoro-6-methoxyphenyl, 3-fluoro-2-methoxyphenyl, 4-fluoro-2-methoxyphenyl, 5-fluoro-2-methoxyphenyl, 3-fluoro-4-methoxyphenyl, 3-fluoro-5-methoxyphenyl, 4-fluoro-3-methoxyphenyl, 2-fluoro-3-nitrophenyl, 2-fluoro-4-nitrophenyl, 2-fluoro-5-nitrophenyl, 2-fluoro-6-nitrophenyl, 3-fluoro-2-nitrophenyl, 4-fluoro-2-nitrophenyl, 5-fluoro-2-nitrophenyl, 3-fluoro-4-nitrophenyl, 3-fluoro-5-nitrophenyl, 4-fluoro-3-nitrophenyl, 2-metoxy-3-nitrophenyl, 2-methoxy-4-nitrophenyl, 2-methoxy-5-nitrophenyl, 2-methoxy-6-nitrophenyl, 3-methoxy-2-nitrophenyl, 4-methoxy-2-nitrophenyl, 5-methoxy-2-nitrophenyl, 3-methoxy-4-nitrophenyl, 3-methoxy-5-nitrophenyl and 4-methoxy-3-nitrophenyl groups. Of which, 3-methoxyphenyl, 4-methoxyphenyl, 4-nitrophenyl and 3,4-dimethoxyphenyl groups are especially preferred.

Preferred examples of the substituted or unsubstituted heteroaryl group include heteroaryl groups such as pyridyl, furanyl, thienyl, pyrrolyl, pyrimidinyl, imidazolyl, isothiazolyl, isoxazolyl, thiazolyl, oxazolyl, thiadiazolyl, pyridazinyl, pyrazinyl and quinolyl groups and, similar to the above-described aryl groups, these heteroaryl groups mono- or di-substituted by a substituent (for example, a C₁₋₈ alkyl group, halogen atom, C₁₋₆ alkoxy group, nitro group, amino group or trihalogenoalkyl group).

As R⁵, R⁶SO₂- is preferred, of which R⁶ representing a phenyl group substituted with a methoxy, nitro or the like group is especially preferred.

R⁷ represents a hydrogen atom or a protecting group. As this protecting group, that readily removable by an acid or alkali is usable. Examples include ethers such as methoxymethyl, ethoxymethyl, propoxymethyl, tetrahydrofuranyl and tetrahydropyranyl, aralkyl groups such as benzyl, p-methoxybenzyl, 2-phenylethyl, 3-phenylpropyl, 4-phenylbutyl and 5-phenylpentyl and silyl groups such as trimethylsilyl and tert-butyldimethylsilyl groups.

Preferred compounds of the present invention represented by the formula (1) each has a hydrogen atom as R¹, a pyridyl or phenyl group as R², a hydrogen atom as R³, an alkyl group such as methyl or isopropyl as R⁴, a substituted phenyl group as R⁵ and CH as A.

No particular limitation is imposed on the salt of the invention compound (1) insofar as it is a pharmaceutically acceptable salt. Examples include (i) salts with a mineral acid such as hydrochloric acid or sulfuric acid, (ii) salts with an organic carboxylic acid such as formic acid, citric acid, acetic acid, trichloroacetic acid, trifluoroacetic acid, fumaric acid or maleic acid, (iii) acid addition salts, for example, salts with sulfonic acid such as methanesulfonic acid, benzenesulfonic acid, p-toluenesulfonic acid, mesitylenesulfonic acid or naphthalenesulfonic acid, (i') salts with an alkali metal such as sodium or potassium, (ii') salts with an alkaline earth metal such as calcium or magnesium, (iii') ammonium salts, (iv') base addition salts, for example, salts with a nitrogen-containing organic base such as trimethylamine, triethylamine, tributylamine, pyridine, N,N-dimethylaniline, N-methylpiperidine, N-methylmorpholine, diethylamine, cyclohexylamine, procaine, dibenzylamine, N-benzyl-β-phenethylamine, 1-ephenamine or N,N'-dibenzylethylenediamine.

The invention compounds (1) or salts thereof may also embrace solvates typified by hydrates.

The invention compounds (1) may also exist in the cis form or trans form. These isomers are embraced in them. In addition, various isomers such as enantiomers, for example, d-(-) isomer and l-(-) isomer and rotational isomers may exist, depending on the kind or combination of the substituents. Any one of these isomers is embraced in the present invention.

The invention compounds (1-A) and (1-B) can be prepared in accordance with Preparation Example I as described below.

### <Preparation Example I>

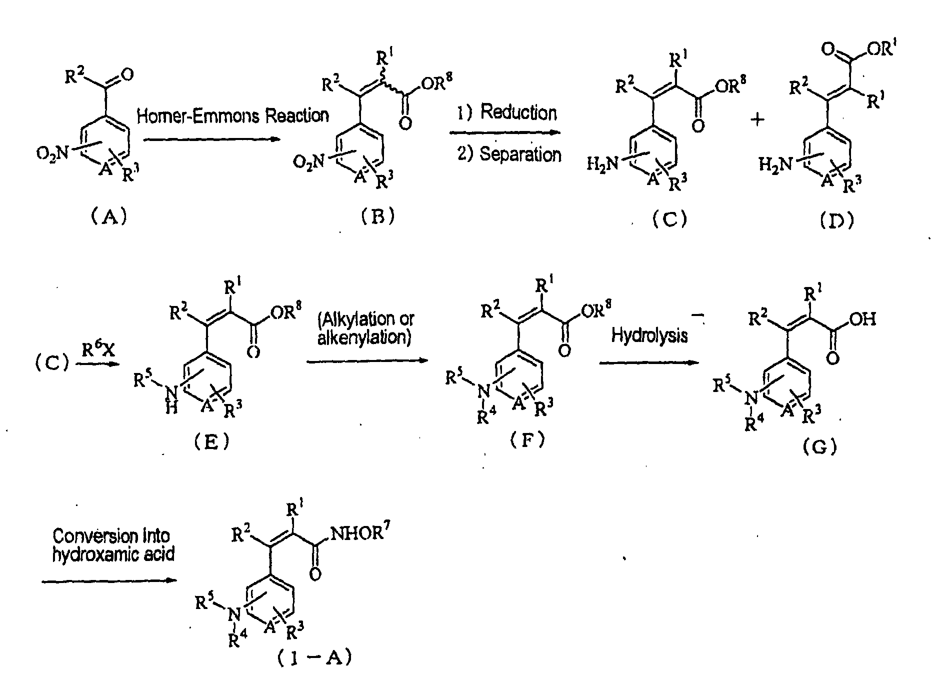

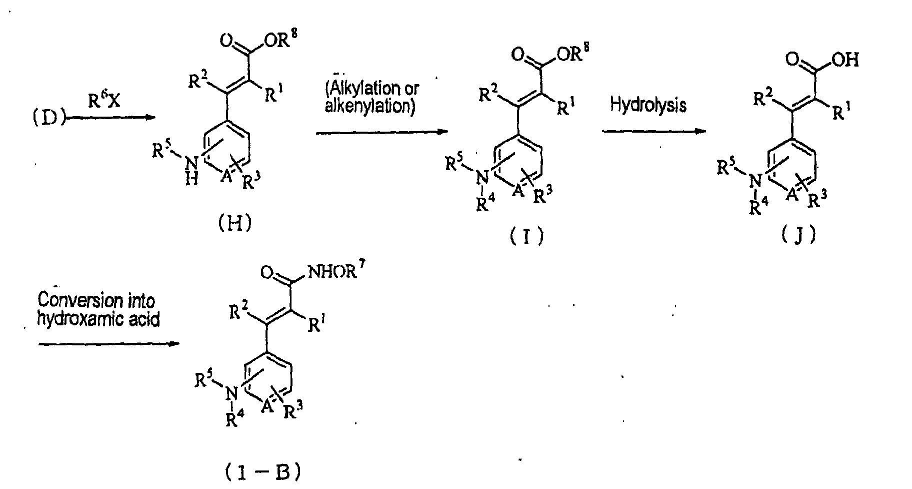

[wherein, R⁸ represents a hydrogen atom or a lower alkyl group, R¹_{,} R², R³, R⁴_{,} R⁵_{,} R⁶_{,} R⁷ and A have the same meanings as described above, and X represents-COOH, -COCl, -NCO, -SO₂Cl, -NCS or -COOCOR⁶] .

Compound (A) employed as a raw material is converted into the corresponding propenoic acid derivative (B) by the Horner-Emmons reaction, followed by reduction and separation of the isomer, whereby Compound (C) and Compound (D) are obtained. Each of these compounds is reacted with R⁶X, whereby each of Compound (E) and Compound (H) is obtained. In order to obtain compound having, as R⁴, a substituted or unsubstituted lower alkyl group or a substituted or unsubstituted lower alkenyl group, Compound (E) and Compound (H) are subjected to alkylation or alkenylation into Compound (F) and Compound (I), followed by hydrolysis into Compound (G) and Compound (J), respectively. They are reacted with a hydroxamic acid converting reagent, whereby the invention compound (I-A) and (I-B) can be prepared, respectively.

Examples of the lower alkyl group represented by R⁸ include C₁₋₆ alkyl groups such as methyl, ethyl, n-propyl, isopropyl, n-butyl, isobutyl, tert-butyl, n-pentyl and n-hexyl groups.

The Horner-Emmons reaction of Compound (A) can be effected in the commonly known method, for example, by reacting Compound (A) with a Horner-Emmons reagent such as trimethyl phosphonoacetate, triethyl phosphonoacetate, triethyl 2-fluorophosphonoacetate or triethyl 2-phosphonopropionate in a solvent, for example, an aromatic hydrocarbon such as benzene, toluene or xylene, an ether such as diethyl ether, tetrahydrofuran, dioxane, monoglyme or diglyme, an alcohol such as methanol, ethanol or propanol, a halogenated hydrocarbon such as methylene chloride, chloroform or carbon tetrachloride or aprotic polar solvent such as acetonitrile, N,N-dimethylformamide or dimethylsulfoxide in the presence of a base such as lithium hydride, potassium hydride, sodium hydride, sodium methoxide, sodium ethoxide, butyl lithium or 1,8-diazabicyclo[5.4.0]undecene (DBU) at 0 to 200°C, preferably at room temperature to 80°C for 10 minutes to 72 hours, preferably 2 to 24 hours.

Reduction of Compound (B) can be effected, for example, by reacting Compound (B) in the presence of a metal such as iron or tin, or chloride or sulfide thereof, or in the coexistence of such a metal and a mineral acid such as hydrochloric acid or sulfuric acid in an alcohol solvent such as methanol, ethanol, propanol or tert-butanol at room temperature to 200°C, preferably at 50°C to 120°C for 10 minutes to 72 hours, preferably 1 to 12 hours; or subjecting Compound (B) to catalytic reduction by using hydrogen or ammonium formate as a hydrogen source, in the presence of palladium-carbon or palladium hydroxide-carbon in a solvent, for example, an alcohol such as methanol or ethanol or acetic acid at room temperature to 120°C, preferably 70°C to 100°C for 30 minutes to 10 hours, preferably 1 to 5 hours.

Compound (C) can be separated from Compound (D) in a manner known per se in the art, for example, column chromatography or crystallization.

The reaction of Compound (C) or Compound (D) can be effected in the presence or absence of an inorganic base such as potassium hydroxide, sodium carbonate or cesium carbonate or an organic base such as pyridine, 4-dimethylaminopyridine, picoline, N,N-dimethylaniline, N-methylmorpholine, dimethylamine, triethylamine or 1,8-diazabicyclo[5.4.0]undecene (DBU) in a solvent, for example, a halogenated hydrocarbon such as methylene chloride, chloroform, carbon tetrachloride or chlorobenzene, an aromatic hydrocarbon such as benzene or toluene, an ether such as tetrahydrofuran, diethyl ether or dioxane, a ketone such as acetone or methyl ethyl ketone or an aprotic polar solvent such as acetonitrile or N,N-dimethylformamide, or ethyl acetate at -30°C to 140°C when X represents -COCl, -NCO, -SO₂Cl or -NCS; in the above-described solvent at -30°C to 100°C when X represents -COOCOR⁶; or in the presence of a condensing agent such as carbonyldiimidazole (CDI) or dicyclohexylcarbodiimide (DCC) in the above-described solvent when X represents -COOH.

The alkylation or alkenylation of Compound (E) or Compound (H) can be conducted, for example, by reacting Compound (E) or Compound (H) with an alkylating agent, e.g., a dialkyl sulfate such as dimethyl sulfate, diethyl sulfate or dipropyl sulfate, an alkyl iodide such as methyl iodide, ethyl iodide, propyl iodide, isopropyl iodide or butyl iodide, an alkyl bromide such as methyl bromide, ethyl bromide, propyl bromide, isopropyl bromide or butyl bromide, an alcohol activated by a sulfonyl group such as methanesulfonyl or p-toluenesulfonyl, or an alkenylating agent such as vinyl bromide or allyl bromide in the presence of an inorganic base such as sodium hydride, potassium hydride, potassium carbonate, sodium carbonate, cesium carbonate, sodium methoxide or sodium ethoxide, or an organic base such as pyridine, picoline, N,N-dimethylaniline, N-methylmorpholine, dimethylamine, triethylamine or 1,8-diazabicyclo[5.4.0]undecene (DBU) in a solvent, e.g., an aromatic hydrocarbon such as benzene or toluene, an ether such as tetrahydrofuran or dioxane or an aprotic polar solvent such as acetonitrile, N-methylpyrrolidone or N,N-dimethylformamide at room temperature to 200°C, preferably at room temperature to 100°C for 10 minutes to 72 hours, preferably 2 to 24 hours.

Hydrolysis of Compound (F) or Compound (I) can be conducted in a manner known per se in the art, for example, by reacting it in the presence of a basic compound such as sodium hydroxide, potassium hydroxide, sodium carbonate or potassium carbonate, a mineral acid such as hydrochloric acid, sulfuric acid or hydrobromic acid, or an organic acid such as p-toluenesulfonic acid, in a solvent, e.g, water, an alcohol such as methanol, ethanol or propanol, an ether such as tetrahydrofuran or dioxane, a ketone such as acetone or methyl ethyl ketone, or acetic acid, or a mixed solvent thereof at room temperature to 140°C, preferably at room temperature to 100°C for 10 minutes to 72 hours, preferably 2 to 24 hours.

Conversion of Compound (G) or Compound (J) to the corresponding hydroxamic acid can be conducted, for example, by reacting Compound (G) or Compound (J) with a hydroxamic acid converting reagent in a solvent, e.g., an aromatic hydrocarbon such as benzene, toluene or xylene, an ether such as diethyl ether, tetrahydrofuran, dioxane, monoglyme or diglyme, a halogenated hydrocarbon such as methylene chloride, chloroform or carbon tetrachloride, or an aprotic polar solvent such as acetonitrile, N,N-dimethylformamide or dimethylsulfoxide in the presence of a condensing agent such as carbonyldiimidazole (CDI), dicyclohexylcarbodiimide (DCC) or 1-ethyl-3-(3-dimethylaminopropyl)carbodiimide-hydrochloride (EDCI) at 0°C to room temperature for 2 to 24 hours. The hydroxamic acid converting reagent and condensing agent are preferably used in amounts of 1.0 to 3.0 moles and 1.0 to 3.0 moles, respectively, per mole of Compound (G) or Compound (J).

Examples of the hydroxamic acid converting reagent include hydroxylamine and protected hydroxylamines such as O-(tert-butyldimethylsilyl)hydroxylamine, O-benzylhydroxylamine, O-(tetrahydro-2H-furan-2-yl)hydroxylamine and O-(tetrahydro-2H-pyran-2-yl)hydroxylamine. Deprotection can be effected by commonly known reaction such as the above-described catalytic reduction or acid treatment.

Condensation between Compound (G) or Compound (J) with a hydroxamic acid converting reagent can be conducted, as well as the above-described reaction, by dissolving Compound (G) or Compound (J) in the above-described solvent, reacting the resulting solution with a chlorocarbonate ester such as methyl chloroformate, -ethyl chloroformate or propyl chloroformate or an acid chloride such as pivaloyl chloride in thepresence of a tertiary amine such as triethylamine or N-methylmorpholine at -30°C to room temperature, preferably at -20°C to 5° C for 5 minutes to 1 hour and then reacting the reaction mixture with the hydroxamic acid converting reagent for 10 minutes to 24 hours, preferably 2 to 8 hours at 0°C to room temperature. In this case, the hydroxamic acid converting reagent and chlorocarbonate ester or acid chloride is preferably used in amounts of 1.0 to 3.0 moles and 1.0 to 1.5 moles per mole of Compound (G) or Compound (J), respectively. When deprotection is necessary, it can be conducted in the above-described manner.

### <Preparation Example II>

The invention compound having as R² a substituted or unsubstituted heteroaryl group wherein the hetero atom is a nitrogen atom or an oxidized nitrogen atom can be prepared, for example, in accordance with the below-described Preparation Example II. Described below is an example of the invention compound having as R² a pyridyl or pyridyl N-oxide group. 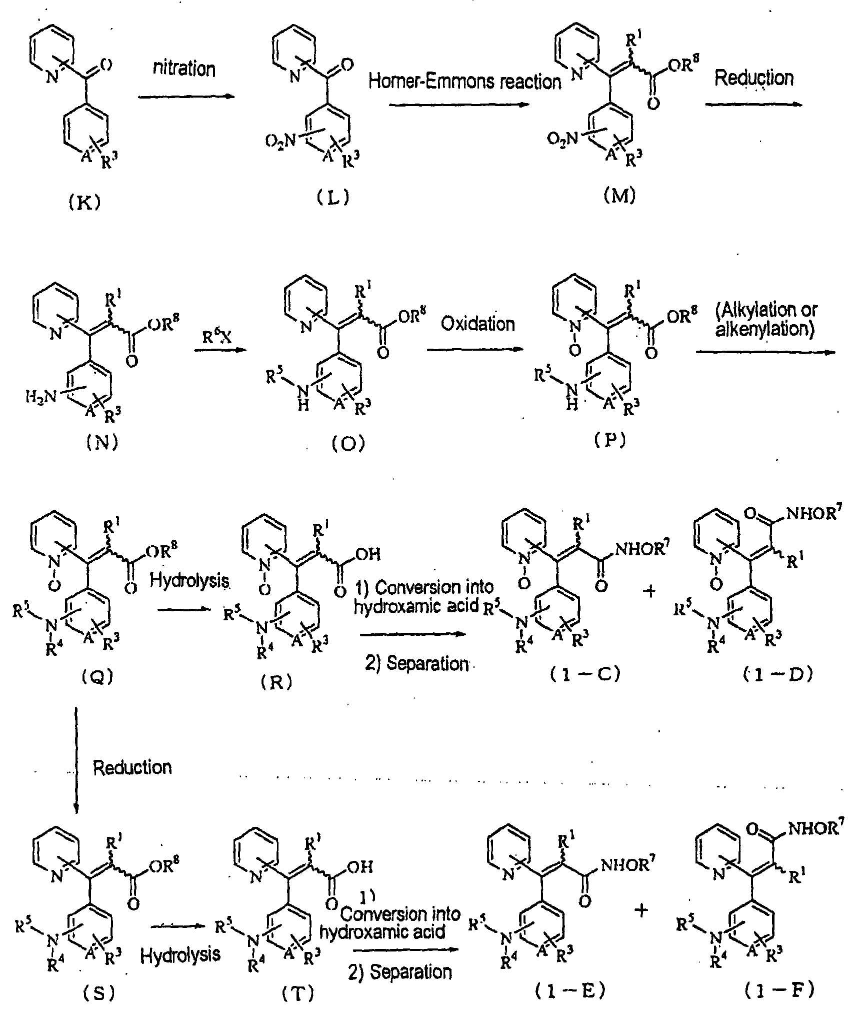 [wherein, R¹, R²_{,} R³_{,} R⁴, R⁵, R⁶, R⁷, R⁸, X and A have the same meanings as described above].

Described specifically, Compound (K) is nitrated into Compound (L), followed by the Horner-Emmons reaction. The resulting Compound (M) is reduced into Compound (N) and it is reacted with R⁶X. The resulting Compound (O) is then oxidized into Compound (P). The resulting compound is then alkylated or alkenylated, if desired, into Compound (Q), followed by hydrolysis. The resulting Compound (R) is reacted with a hydroxamic acid converting reagent, followed by separation, whereby the invention Compound (1-C) and Compound (1-D) can be prepared.

The invention Compounds (1E) and (1-F) are available by subjecting Compound (Q) to reduction, hydrolyzing the resulting Compound (S) into Compound (T), reacting it with a hydroxamic acid converting reagent and then separating the reaction mixture.

The nitration of Compound (K) can be effected in a manner known per se in the art. Examples of a nitration agent usable here include an acid mixture of nitric acid or a nitrate with sulfuric acid, and acetyl nitrate. The reaction can be conducted by adding Compound (K) to, for example, the acid mixture and reacting them at -10°C to 80°C for 5 minutes to 5 hours. As the acid mixture, sulfuric acid and nitric acid can be added each in an equimolar amount to a large excess amount relative'to the compound.

Although oxidation of Compound (O) can be effected in a manner known per se in the art, it can be preferably conducted by reacting in the presence of an organic peroxide such as m-chlorobenzoic acid or magnesium monophthalate or hydrogen peroxide in a solvent, e.g., an aromatic hydrocarbon such as benzene, toluene or xylene, or a halogenated hydrocarbon such as methylene chloride, chloroform or carbon tetrachloride at -30°C to 100°C, preferably -10°C to room temperature for 10 minutes to 72 hours, preferably 1 to 12 hours.

The N-oxide reduction from Compound (Q) to Compound (S) can be conducted in a similar manner to the reduction of the nitro group in Compound (M).

Reduction, Horner-Emmons reaction, reaction with R⁶X, hydrolysis, conversion into hydroxamic acid, alkylation and alkenylation may each be conducted in a similar manner to that employed in Preparation Example I.

The compounds according to the present invention can be isolated by the purifying method ordinarily employed in organic synthetic chemistry, for example, filtration, washing, drying, recrystallization or various chromatographies. They are provided in the form of salts, free carboxylic acids or free amines according to conditions for isolation and purification. These compounds are mutually converted, if desired, to prepare the compounds according to the present invention in the intended form.

Since the compounds (1) according to the present invention or the salts thereof have excellent TACE inhibitory action as described later in Examples, they are useful as a medicament for prevention and/or treatment of various diseases resulting from excessive extracellular release of TNF-α such as septicemia, rheumatoid arthritis, osteoarthritis, infectious diseases, autoimmune diseases, malignant neoplasm, collagenosis, chronic ulcerative colitis, MOF and insulin-independent diabetes.

When the compounds (1) according to the present invention or salts thereof are used as a medicament, they can be formulated into compositions together with a pharmaceutically acceptable carrier for parenteral administration such as injection administration or intrarectal administration, or for oral administration in a solid or liquid form.

Examples of the preparation form of injection include solutions in pharmaceutically acceptable sterile water, non-aqueous solutions, suspensions and emulsions. Suitable examples of non-aqueous carriers, diluents, solvents or vehicles include propylene glycol, polyethylene glycol, vegetable oils such as olive oil, and injectable organic esters such as ethyl oleate. In such compositions, may be incorporated auxiliaries such as an antiseptic, humectant, emulsifier and dispersing agent. These compositions can be sterilized by filtration through a bacterial filter, or mixing a sterilizing agent right before use or mixing a sterilizing agent in the form of a sterile solid composition soluble in another medium sterilely injectable.

Examples of solid preparations for oral administration include capsules, tablets, pills, powder and granules. Upon formulation of such a solid preparation, the compound according to the present invention is mixed with at least one inert diluent, for example, sucrose, lactose or starch. In the general formulation of the solid preparation, other additives, for example, a lubricant (such as magnesium stearate) may be incorporated into this preparation in addition to the inert diluent. In the cases of the capsules, tablets and pills, a buffer may also be additionally used. The tablets and pills may be subjected to enteric coating.

Examples of liquid preparations for oral administration include pharmaceutically acceptable emulsions, solutions, suspensions, syrups and elixirs containing an inert diluent commonly used by those skilled in the art, for example, water. In such compositions, may also be incorporated auxiliaries, for example, a humectant, emulsifier, suspending agent, sweetener, taste corrigent and smell corrigent. A preparation for intrarectal administration may preferably contain an excipient, for example, cacao butter or suppository wax, in addition to the compound according to the present invention.

The dose of the compound (1) or salt thereof according to the present invention depends on the properties of a compound administered, the administration route thereof, desired treatment time, and other factors. However, it is preferred to administer the compound in a dose of generally about 0.1 to 100 mg/kg, particularly, about .0.1 to 50 mg/kg a day. This amount of the compound may also be administered in 2 to 4 portions a day.

### Examples

The present invention will hereinafter be described in detail by Examples.

### Referential Example 1 (1)

### Synthesis of ethyl E,Z-3-(3-nitrophenyl)-3-phenylpropenoate (Compound 1)

To a suspension of 6.30 g (60% in oil) of sodium hydride in 100 mL of tetrahydrofuran was added dropwise a 100 mL tetrahydrofuran solution of 29.7 g of triethyl phosphonoacetate under ice cooling. After stirring for 1 hour at the temperature raised back to room temperature, 15.0 g of 3-nitrobenzophenone was added and the mixture was stirred for 2 hours. Tetrahydrofuran was distilled off under reduced pressure. To the residue were added 300 mL of water and 500 mL of ethyl acetate. The organic layer was separated and the water layer was extracted three times, each with 100 mL of ethyl acetate. All the organic layers were collected, dried over anhydrous magnesium sulfate and distilled under reduced pressure to remove the solvent. The residue was subjected to chromatography on a silica gel column (200 cc, hexane:ethyl acetate = 4:1 to 2:1), whereby 14.5 g of a mixture of the title compound was obtained as a pale yellow oil.

### Referential Examples 1 (2) to (6)

In a similar manner to Referential Example 1 (1), the following Compounds 2 to 6 were synthesized.

### Referential Example 1 (2)

### Ethyl E,Z-3-(4-nitrophenyl)-3-phenylpropenoate (Compound 2)

### Referential Example 1 (3)

### Ethyl E,Z-3-(3-nitrophenyl)-3-(3-pyridyl)propenoate (Compound 3)

### Referential Example 1 (4)

### Ethyl 3,3-bis(3-nitrophenyl)propenoate (Compound 4)

Melting point: 99 to 100°C

### Referential Example 1 (5)

### Ethyl 3,3-bis(3-nitrophenyl)-2-fluoropropenoate (Compound 5)

### Referential Example 1 (6)

### Ethyl 3, 3-bis (3-nitrophenyl)-2-methylpropenoate (Compound 6)

### Referential Example 2 (1)

### Synthesis of ethyl E-3-(3-aminophenyl)-3-phenylpropenoate (Compound 7) and ethyl Z-3-(3-aminophenyl)-3-phenylpropenoate (Compound 8)

After 8.20 g of iron powder was suspended in 120 mL of water, 1.4 mL of 36% hydrochloric acid was added dropwise to the resulting suspension at room temperature. After stirring for 1 hour, a solution of 14.5 g of ethyl E, Z-3- (3-nitrophenyl)-3-phenylpropenoate in 40 mL of ethanol was added. The resulting mixture was stirred under heat at 80°C. Three hours later, the temperature was returned to room temperature and the insoluble matters were filtered off. The filtrate was extracted three times, each with 100 mL of ethyl acetate. All the organic layers were combined, washed with water, dried over anhydrous magnesium sulfate and distilled under reduced pressure to remove the solvent. The residue was subjected to chromatography on a silica gel column (500 cc, hexane:ethyl acetate = 8:1 to 2:1) to separate the first eluate fraction of ethyl E-3-(3-aminopheny)-3-phenylpropenoate from the subsequent eluate fraction of ethyl 2-3-(3-aminophenyl)-3-phenylpropenoate, whereby 1.10 g of (Compound 7) and 3.50 g of (Compound 8) were obtained. In addition, 3.70 g of an isomer mixture not separable by chromatography was obtained.

### Compound 7:

Appearance: Yellow oil
¹H-NMR (CDCl₃) δ:
1.10(3H,t,J=8Hz), '3.64(2H,brs), 4.03(2H,q,J=8Hz), 6.33(1H,s), 6.55-6.56(lH,m), 6.67(1H,dd,J=8Hz,3Hz), 6.71-6.73(1H,m), 7.10(1H,t,J=8Hz), 7.35-7.37(5H,m)

### Compound 8:

Appearance: Pale yellow crystal
Melting point: 81 to 82°C
¹H-NMR (CDCl₃) δ:
1.14(3H,t,J=8Hz), 3.64(2H,brs), 4.07(2H,q,J=8Hz), 6.32(1H,s), 6.52(1H,brs), 6.62(1H,d,J=8Hz), 6.69-6.71(1H,m), 7.17(1H,t,J=8Hz) 7.31-7.36(5H,m)

### Referential Examples 2 (2) to (4)

In a similar manner to Referential Example 2 (1), the following Compounds 9 to 11 were synthesized.

### Referential Example 2 (2)

### Ethyl E,Z-3-(4-aminophenyl)-3-phenylpropenoate (Compound 9)

### Referential Example 2 (3)

### Ethyl E,Z-3-(3-aminophenyl)-3- (3-pyridyl)propenoate (Compound 10)

### Referential Example 2 (4)

### Ethyl 3,3-bis (3-aminophenyl)propenoate (Compound 11)

### Example 1 (1)

### Synthesis of ethyl Z-3-[3-(4-methoxybenzenesulfonylamino)phenyl]-3-phenylpropenoate (Compound 12)

In 3 mL of pyridine was dissolved 267 mg of Z-3-(3-aminophenyl)-3-phenylpropenoate. To the resulting solution was added 248 mg of 4-methoxybenzenesulfonyl chloride, followed by stirring. After one hour and 30 minutes, the reaction mixture was poured into 5 mL of 5% hydrochloric acid and 15 mL of ethyl acetate. The organic layer was separated, washed with water, dried over anhydrous magnesium sulfate and distilled under reduced pressure to remove the solvent. The solid thus precipitated was dispersed in diisopropyl ether, collected by filtration and dried, whereby 397 mg of the title compound was obtained as a pale red solid.
¹H-NMR (CDCl₃) δ:
1.14(3H,t,J=8Hz), 3.82(3H,s), 4.03(2H,q,J=8Hz), 6.30(1H,s) 6.82-7.38(11H,m) 7.62(2H,d,J=9Hz)

### Example 1 (2)

In a similar manner to Example 1 (1), the following Compound (13) was synthesized.

### Example 1 (2)

### Ethyl 3,3-bis[3-(4-methoxybenzenesulfonylamino)phenyl]propenoate (Compound 13)

¹H-NMR (CDCl₃) δ:
1.07(3H,t,J=7Hz), 3.80(3H,s), 3.84(3H,s), 4.00(2H,q,J=7Hz), 6.21(1H, 6.81-7.25(12H,m), 7.61(2H,d,J=9Hz), 7.67(2H,d,J=9Hz)

### Example 2 (1)

### Synthesis of Z-3-[3-(4-methoxybenzenesulfonylamino)phenyl]-3-phenylpropenoic acid (Compound 14)

In 10 mL of methanol was dissolved 390 mg of ethyl Z-3-[3-(4-methoxybenzenesulfonylamino)phenyl]-3-phenylpropenoate. To the resulting solution was added 6 mL of 5% sodium hydroxide, followed by stirring under heat at 65°C. After 2 hours and 30 minutes, methanol was distilled off under reduced pressure. The residue was adjusted to pH 1 with 5% hydrochloric acid and extracted three times, each with 15 mL of ethyl acetate. The organic layers were combined, washed with water, dried over anhydrous magnesium sulfate and distilled under reduced pressure to remove the solvent. The solid thus precipitated was dispersed in diisopropyl ether, collected by filtration and dried, whereby 300 mg of the title compound was obtained as a pale brown solid.
¹H-NMR (CDCl₃) δ:
3.81(3H,s), 6.32(1H,s), 6.82-7.39(11H,m), 7.62(2H,d,J=9Hz)

### Example 2 (2)

In a similar manner to Example 2 (1), the following Compound 15 was synthesized.

### Example 2 (2)

### 3,3-bis [3-(4-Methoxybenzenesulfonylamino)phenyl]propenoic acid (Compound 15)

¹H-NMR (DMSO-d₆) δ:
3.78(3H,s), 3.80(3H,s), 6.16(1H,s) 6.74(1H,d,J=8Hz), 6.78(1H,d,J=8Hz) , 6.84(1H,s), 6.94(1H,s), 6.78-7.05(6H,m), 7.17(1H,t,J=8H 7.22(1H,t,J=8Hz), 7.58(4H,t,J=8Hz), . 10.08(1H,s), 10.21(1H,s), 12.22(1H,brs)

### Example 3 (1)

### Synthesis of Z-3-[3- (4-methoxybenzenesulfonylamino)phenyl]-3-phenylpropenohydroxamic acid (Compound 16)

In 3 mL of N,N-dimethylformamide was dissolved 139 mg of Z-3-[3-(4-methoxybenzenesulfonylamino)phenyl]-3-phenylpropenoic acid. To the resulting solution were successively added 70 mg of 1-hydroxybenzotriazole, 100 mg of 1-ethyl-3-(3-dimethylaminopropyl)carbodiimide hydrochloride, 53 mg of N-methylmorpholine and 100 mg of o-(tert-butyldimethylsilyl)hydroxylamine. After stirring for 17 hours, the reaction mixture was poured into 5 mL of water and 15 mL of ethyl acetate and the mixture was stirred for 30 minutes. The reaction mixture was then extracted three times, each with 15 mL of ethyl acetate. The organic layer was washed three times, each with 10 mL of water, dried over anhydrous magnesium sulfate and distilled under reduced pressure to remove the solvent. The solid thus precipitated was dispersed in diisopropyl ether and collected by filtration, whereby 76 mg of pale brown powder was obtained.
Appearance: Pale brown powder
¹H-NMR (DMSO-d₆) δ:
3.81(3H,s), 6.26(1H,s), 6.81-6.85(2H,m), 7.02-7.05(5H,m), 7'.18-7.21(lH,m), 7.30-7.36(3H,m), 7.60(2H,d,J=8Hz), 8.82(1H,brs), 10.00(1H,brs), 10.58(1H,brs)

### Example 3 (2) to (35)

In a similar manner to Example 3 (1), the following Compounds 17 to 50 were synthesized.

### Example 3 (2)

### Z-3-[3-(4-Bromobenzenesulfonylamino)phenyl]-3-phenylpropenohydroxamic acid (Compound 17)

Appearance: Pale orange powder
¹H-NMR (DMSO-d₆) δ:
6.28(1H,s), 6.81(1H,s), 6.88(1H,d,J=8Hz), 7.02-7.07(3H,m), 7.23(1H,t,J=8Hz), 7.32-7.37(3H,m), 7.58(2H,d,J=9Hz), 7.74(2H,d,J=9Hz), 8.84(1H,br 10.24(1H,brs), 10.61 (1H,brs)

### Example 3 (3)

### Z-3-[3-(4-Fluorobenzenesulfonylamino)phenyl]-3-phenylpropenohydroxamic acid (Compound 18)

Appearance: Pale brown powder
¹H-NMR (DMSO-d₆) δ:
6.26(1H,s), 6.27-7.73(13H,m), 8.82(1H,brs), 10.00(1H,brs), 10.60(1H,brs)

### Example 3 (4)

### Z-3-[3-(3-Nitrobenzenesulfonylamino)phenyl]-3-phenylpropenohydroxamic acid (Compound 19)

Appearance: Pale orange powder
¹H-NMR (DMSO-d₆) δ:
6.26(1H,s), 6.81(1H,s), 6.89(1H,d,J=8Hz), 7.07(2H,d,J=8Hz), 7.67(1H,d,J=8Hz), 7.21-7.36(4H,m), 7.84(1H,t,J=8Hz), 8.05(1H,d,J=8Hz), 8.41(1H,s), 8.46(1H,d,J=9Hz), 8.81(1H,brs) 10.48(1H,brs) 10.60(1H,brs)

### Example 3 (5)

### Z-3-[3-(4-Trifluoromethoxybenzenesulfonylamino)phenyl]-3-phenylpropenohydroxamic acid (Compound 20)

Appearance: Colorless powder
¹H-NMR (DMSO-d₆) δ:
6.27(1H,s) 6.84-7.31(9H,m), 7.52(2H,d,J=8Hz), 7.79(2H,d,J=8Hz), 8.82(1H,brs), 10.29(1H,brs), 10.60(1H,brs)

### Example 3 (6)

### Z-3-[3-(4-Butoxybenzenesulfonylamino)phenyl]-3-phenylpropenohydroxamic acid (Compound 21)

Appearance: Colorless powder
¹H-NMR (DMSO-d₆) δ:
0.93(3H,t,J=7Hz), 1.41-1.46(2H,m), 1.68-1.99(2H,m), 4.01(2H,t,J=7Hz), 6.27(1H,s), 6.80(1H,s) 6.84(1H,d,J=8Hz), 7.00-7.06(5H,m), 7.20(1H,t,J=8Hz), 7.30-7.35(3H,m), 7.58(2H,d,J=9Hz), 8.82(1H.brs), 10.00(1H,brs), 10.58(1R,brs)

### Example 3 (7)

### Z-3- [3-(4-Acetoamidobenzenesulfonylamino)phenyl]-3-phenylpropenohydroxamic acid (Compound 22)

Appearance: Colorless powder
¹H-NMR (DMSO-d₆) δ:
2.50(3H,s), 6.26(1H,s), 6.82-7.68(13H,m), 8.82(1H,brs), 10.00(2H,brs), 10.30(1H,brs)

### Example 3 (8)

### Z-3-[3-(Phenylmethylsulfonylamino)phenyl]-3-phenylpropenohydroxamic acid (Compound 23)

Appearance: Colorless powder
¹H-NMR (DMSO-d₆) δ:
4.46(2H,s), 6.33(1H,s), 6.84(1H,d,J=8Hz), 7.10-7.40(13H,m), 8.83(1H,brs), 9.84(1H,brs), 10.62(1H,brs)

### Example 3 (9)

### Z-3- [3- (3, 4-Dimethoxybenzenesulfonylamino) phenyl] -3-phenylpropenohydroxamic acid (Compound 24)

Appearance: Colorless powder
¹H-NMR (DMSO-d₆) δ:
3.67(3H,s), 3.82(3H,s), 6.27(1H,s) 6.82(1H,s), 6.86(1H,d,J=8Hz), 7.02-7.37(10H,m), 8.81(1H,brs), 9.95(1H,brs), 10.58(1H,brs)

### Example 3 (10)

### Z-3-[3-(2,4,6-Triisopropylbenzenesulfonylamino)phenyl]-3-phenylpropenohydroxamic acid (Compound 25)

Appearance: Colorless powder
¹H-NMR (DMSO-d₆) δ:
1.07(12H,d,J=7Hz), 1.18(6H,d,J=7Hz), 2.89(1H,septet,J=7Hz), 4.11(2H,septet,J=7Hz),6.23(1H,s), 6.73(1H,s), 6.80-7.32(10H,m), 8.80(1H,brs), 10.11(1H,brs), 10.57(1H,brs)

### Example 3 (11)

### Z-3-[3-(1-Piperidinesulfonylamino)phenyl]-3-phenylpropenohydroxamic acid (Compound 26)

Appearance: Colorless crystalline powder
Melting point: 158 to 159°C
¹H-NMR (DMSO-d₆) δ:
1.36(6H,s), 3.01(4H,s), 6.28(1H,s), 6.88(1H,d,J=7Hz), 6.93(1H,s), 7.13-7.28(4H,m), 7.36(3H,s), 8.80(1H,s), 9.79(1H,brs), 10.57(1H,brs

### Example 3 (12)

### Z-3- [3- (1-Naphthalenesulfonylamino)phenyl] -3-phenylpropenohydroxamic acid (Compound 27)

Appearance: Colorless crystalline powder
Melting point: 160 to 163°C
¹H-NMR (DMSO-d₆) b:
6.20(1H,s), 6.75(2H,t,J=8Hz), 6.88-6.95(3H,m), 7.09(1H,t,J=8Hz), 7.25-7.35(3H,m), 7.56(1H,t,J=8Hz), 7.63-7.70(2H,m), 8.07(2H,d,J=8Hz), 8.07(1H,d,J=8Hz), 8.21(1H,d,J=8Hz), 8.79(1H,brs), 10.56(2H,brs)

### Example 3 (13)

### Z-3-[3-(2-Naphthalenesulfonylamino)phenyl]-3-phenylpropenohydroxamic acid (Compound 28)

Appearance: Colorless powder
¹H-NMR (DMSO-d₆) δ:
6.25(1H,s), 6.80(1H,d,J=8Hz), 6.89(1H,s), 6.92(2H,d,J=8Hz), 7.05-7.28(4H,m), 7.65-7.74(3H,m), 8.02-8.12(3H,m), 8.34(2H,d,J=7Hz), 8.83(1H,brs) 10.27(1H,brs), 10.59(1H,brs)

### Example 3 (14)

### Z-3-[3-(8-Quinolinesulfonylamino)phenyl]-3-phenylpropenoic acid (Compound 29)

Appearance: Pale yellow powder
¹H-NMR (DMSO-d₆) δ:
6.19(1H, s), 6.71(1H,d,J=8Hz), 6.75-7.34(7H,m), 7.65-7.70(2H,m), 8.24-8.29(2H,m), 8.50(1H,dd,J=2Hz,4Hz), 8.79(1H,brs), 8.84(1H,s), 9.06(1H,dd,J=2Hz,4Hz), 10.01(1H,brs 10.54(1H,brs)

### Example 3 (15)

### Z-3- [3-[5-(Dimethylamino)-1-naphthalenesulfonylamino)phenyl]-3-phenylpropenohydroxamic acid (Compound 30)

Appearance: Pale yellow crystalline powder
Melting point: 123 to 125°C
¹H-NMR (DMSO-d₆) δ:
2.81(6H,s), 6.21(1H,S), 6.75(1H,d,J=8Hz), 6.77(1H,s), 6.92-6.96(3H,m), 7.09-7.12(1H, 7.22-7.35(4H,m), 7.51-7.58(2H,m), 8.05(1H,d,J=7Hz), 8.31-8.34(1H,m), 8.43(1H,d,J=9Hz), 8.79(1H,brs), 10.53(2H,brs)

### Example 3 (16)

### Z-3-[3-(1-Propanesulfonylamino)phenyl]-3-phenylpropenohydroxamic acid (Compound 31)

Appearance: Pale yellow powder
¹H-NMR (DMSO-d₆) δ:
0.93(3H,t,J=7Hz), 1.63-1.67(2H,m), 3.06(2H,t,J=7Hz), 6.31(1H,s), 6.86(1H,d,J=8Hz 7.02-7.37(8H,m), 8.81(1H,brs), 9.75(1H,brs), 10.60(1H,brs)

### Example 3 (17)

### E-3-(3-(4-Methoxybenzenesulfonylamino)phenyl]-3-phenylpropenohydroxamic acid (Compound 32)

Appearance: Colorless powder
¹H-NMR (DMSO-d₆) δ:
3.82(3H,s), 6.15(1H,s), 6.88(1H,s), 6.94(1H,d,J=8Hz), 7.03-7.05(5H/m), 7.22(1H,t,J=8Hz), 7.34-7.35(3H,m), 7.57(2H,d,J=8Hz), 8.82(1H,brs), 10.14(1H,brs), 10.59(1H,brs)

### Example 3 (18)

### E-3-[3-(4-Bromobenzenesulfonylamino)phenyl]-3-phenyl-2-propenohydroxamic acid (Compound 33)

Appearance: Colorless powder
¹H-NMR (DMSO-d₆) δ:
6.17(1H,s), 6.81(1H,S), 6.88(1H,d,J=8Hz), 7.02-7.07(4H,m), 7.26(1H,t,J=8Hz), 7.34-7.35(2H,m), 7.54(2H,d,J=9Hz), 7.76(2H,d,J=9Hz), 8.83(1H,brs), 10.35(1H,brs 10.60(1H,brs)

### Example 3 (19)

### E-3-[3-(3-Nitrobenzenesulfonylamino)phenyl]-3-phenylpropenohydroxamic acid (Compound 34)

Appearance: Colorless powder
¹H-NMR (DMSO-d₆) δ:
6.18(1 6.78(1H,s), 6.99-7.18(4H,m), 7.26-7.30(4H,m), 7.84(1H,t,J=8Hz), 8.00(1H,d,J=8Hz), 8.38(1H,s), 8.47(1H,d,J=8Hz), 8.82(1H,brs), 10.58(2H,brs)

### Example 3 (20)

### Z-3-[4-(4-Nitrobenzenesulfonylamino)phenyl]-3-phenylpropenohydroxamic acid (Compound 35)

Appearance: Pale brown crystalline powder
Melting point: 146 to 148°C
¹H-NMR (DMSO-d₆) δ:
6.20(1H,s), 7.02-7.34(9H,m), 8.01(2H,d,J=9Hz), 8.39(2H,d,J=9Hz), 8.80(1H,brs), 10.54(1H,brs), 10.68 (1H,brs)

### Example 3 (21)

### Z-3- [2-Chloro-5-(2-naphthalenesulfonylamino)phenyl]-3-phenylpropenohydroxamic acid (Compound 36)

Appearance: Colorless crystalline powder
Melting point: 173 to 175°C
¹H-NMR (DMSO-d₆) δ:
6.41(1H,s), 6.80(1H,d,J=9Hz), 6.93(1H,d,J=3Hz), 7.06-7.10(3H,m), 7.24-7.28(3H,m), 7.64-7.73(3H,m), 8.03(1H,d,J=8Hz), 8.08(1H,d,J=8Hz), 8.13(1H,d,J=8Hz), 8.37(1H,s), 8.91(1R,brs), 10.40(1H,brs), 10.7,0(1H,brs)

### Example 3 (22)

### Z-3-[2-Chloro-5-(3,4-dimethoxybenzenesulfonylamino)phenyl]-3-phenylpropenohydroxamic acid (Compound 37)

Appearance: Colorless crystalline powder
Melting point: 127 to 130°C
¹H-NMR (DMSO-d₆) δ:
3.68(3H,s), 3.81(3H,s), 6.44(1H,s), 6.86(1H,d,J=3Hz), 6.98-7.08(4H/m), 7.18-7.37(6H,m), 8.89(1H,brs), 10.11(1H 10.69(1H,brs)

### Example 3 (23)

### 3,3-bis[3-(4-Methoxybenzenesulfonylamino)phenyl]propenohydroxamic acid (Compound 38)

Appearance: Colorless powder
¹H-NMR (DMSO-d₆) δ: 3.78(3H,s), 3.81(3H,s), 6.12(1H,s), 6.68(1H,d,J=8Hz), 6.75(1H,d,J=7Hz), 6.83(1H,s), 6.96-7.05(6H,m), 7.14-7.20(2H,m), 7.59-7.62 (5H,m), 8.83(1H,brs), 10.06(1H,brs), 10.21(1H,brs), 10.63(1H,brs)

### Example 3 (24)

### 3, 3-bis [3- (4-Methoxybenzoylamino) phenyl] propenohydroxamic acid (Compound 39)

Appearance: Colorless crystalline powder
Melting point: 198 to 200°C
¹H-NMR (DMSO-d₆) δ:
3.83(6H,s), 6.25(1H,s), 6.93(1H,d,J=8Hz), 6.97(1H,d,J=8Hz), 7.04(4H,d,J=8Hz), 7.30-7.36(2H,m), 7.56(1H,s), 7.68(1H,s 7.82-7.85(2H,m), 7.93-7.95(4H,m), 8.83(1H,brs), 10.08(1H,brs), 10.13(1H,brs), 10.63(1H,brs)

### Example 3 (25)

### 3, 3-bis [3(4-Bromobenzenesulfonylamino)phenyl]propenohydroxamic acid (Compound 40)

Appearance: Colorless crystalline powder
Melting point: 137 to 141°C
¹H-NMR (DMSO-d₆) δ:
6.16(1H,S), 6.75(2H,dd,J=2Hz,8Hz), 6.86(1H,s), 6.88.(1H,s), 7.02(2H,d,J=8Hz), 7.20'(2''H,dd,J=2Hz,8Hz), 7.57-7.60(4H,m), 7.70(2H,d,J=8Hz), 7.75(2H, d, J=8Hz), 8 . 86 (1H, brs) , 10.37(2H,brs), ), 10.65(1H,brs)

### Example 3 (26)

### 3, 3-bis [3- (2-Nitrobenzenesulfonylamino)phenyl]propenohydroxamic acid (Compound 41)

Appearance: Colorless powder
¹H-NMR (DMSO-d₆) δ:
6.16(1H,s 6.76-6.80(2H,m), 6.89(1H,s), 6.93(1H,s), 7.07(2H,d,J=8Hz), 7.20-7.24(2H,m), 7.71-7.85(6H,m), 7.92-7.97(2H,m), 8.84(1H,brs), 10.65(1H,brs), 10.68(2H,brs)

### Example 3 (27)

### 3,3-bis[3-(3-Nitrobenzenesulfonylamino)phenyl]propenohydroxamic acid (Compound 42)

Appearance: Colorless powder
¹H-NMR (DMSO-d₆) δ: 6.15(1H,s), 6.74-7.21(8H,m), 7.77-7.86(2H,m), 7.99-8.05(2H,m), 8.44-8.46(4H,m), 8.81(1H,brs), 10.51(2H,brs), 10.62(1H,brs)

### Example 3 (28)

### 3,3-bis[3-(3-Phenylmethylsulfonyl)amino]phenyl]propenohydroxamic acid (Compound 43)

Appearance: Colorless powder
¹H-NMR (DMSO-d₆) δ: 4.40(2H,s), 4.45(2H,s), 6.28(1H,s), 6.86(2H,d,J=8Hz), 7.02-7.34(10H,m), 7:99-8.05(2H,m), 8.44-8.46(4H,m), 8.85(1H,brs), 9.85(2H,brs), 10.69(1H,brs)

### Example 3 (29)

### 3, 3-bis [3- (3,4-Dimethoxybenzenesulfonylamino)phenyl]propenohydroxamic acid (Compound 44)

Appearance: Colorless crystalline powder
Melting point: 132 to 135°C
¹H-NMR (DMSO-d₆) δ: 3.68(3H,s), 3.73(3H,s), 3.78(3H,s), 3.80(3H,s), 6.12(1H,s) 6.62-7.33(14H,m), 8.81(1H,brs), 10.00(1H,brs), 10.14(1H,brs), 10.63(1H,brs)

### Example 3 (30)

### 3,3-bis[3-(2-Naphthalenesulfonylamino)phenyl]propenohydroxamic acid (Compound 45)

Appearance: Pale orange powder
Melting point: 128 to 131°C
¹H-NMR (DMSO-d₆) δ:
6.09(1H,s), 6.44(1H,d,J=8Hz), 6.62(1H,d,J=8Hz), 6.61-6.87(2H,m), 7.01-7.07(4H,m), 7.58-7.70(6H,m), 7.94-8.10(6H,m), 8.34(1H,s), 8.38(1H,s), 8.82(1H,brs), 10.32(2H,brs), 10.62(1H,brs)

### Example 3 (31)

### 3, 3-bis [3- (8-Quinolinesulfonylamino)phenyl]propenohydroxamic acid (Compound 46)

Appearance: Colorless crystalline powder
Melting point: 152 to 156°C
¹H-NMR (DMSO-d₆) δ:
5.91(1H,s), 6.19(1H,d,J=8Hz), 6.47(1H,d,J=8Hz), 6.69(1H,s), 6.82-6.89(2H,m), 6.95-6.98(3H,m), 7.57-7.69(4H,m), 8.20-8.25(4H,m), 8 . 44 (1H, dd, J=2Hz, 8Hz) , 9.02(2H,t,J=4Hz), 8.50(1H,dd,J=2Hz,8Hz), 8.76(1H,brs), 10.05(2H,brs), 10.52(1H,brs)

### Example 3 (32)

### 2-Fluoro-3,3-bis[3-(4-methoxybenzenesulfonylamino)phenyl]propenohydroxamic acid (Compound 47)

Appearance: Colorless powder
¹H-NMR (DMSO-d₆) δ:
3.78(6H,s), 6.40(1H,d,J=8Hz), 6.79(1H,d,J=7Hz), 6.89-7.25(8H,m), 7.52-7.63(6H,m), 10.11(1H,brs), 10.32(2H,s), 11.25(1H,S)

### Example 3 (33)

### 2-Fluoro-3,3-bis[3-(2-nitrobenzenesulfonylamino)phenyl]propenohydroxamic acid (Compound 48)

Appearance: Colorless powder
1H-NMR (DMSO-d₆) δ:
6.91-7.30(8H,m), 7.67-7.95(8H,m), 9.20(1H,s), 10.79(1H,brs), 11.25(1H,s)

### Example 3 (34)

### 2-Fluoro-3,3-bis[3-(8-quinolinesulfonylamino)phenyl]propenohydroxamic acid (Compound 49)

Appearance: Colorless crystalline powder
Melting point: 150 to 153°C
¹H-NMR (DMSO-d₆) δ:
6.54(1H,d,J=8Hz), 6.88-7.01(6H,m), 7.57-7.72(5H,m), 8.18-8.28(4H,m), 8.47-8.53(2H,m), 9.05(1H,dd,J=2Hz,4Hz), 9.09(1H,dd,J=2Hz,4Hz), 9.16(1H,brs), 10.10(1H,brs), 10.20(1H,brs), 11.17(1H,s)

### Example 3 (35)

### 2-Methyl-3,3-bis[3-(4-methoxybenzenesulfonylamino)phenyl]-propenohydroxamic acid (Compound 50)

Appearance: Colorless powder
¹H-NMR (DMSO-d₆) δ: 2.50(3H,s), 3.77(3H,s), 3.78(3,H,s), 6.55(1H,s), 6.59(1H,d,J=8Hz), 6.80-7.00(9H,m), 7.06(1H,t,J=8Hz), 7.20(1H,t,J=8Hz), 7.50(2H,d,J=8Hz), 7.60(2H,d,J=8Hz), 8.70(1H,brs), 10.02(1H,brs), 10.14(1H,brs), 10.45(1H,brs)

### Example 4 (1)

### Synthesis of Z-3-[3-(4-methoxybenzoylamino)phenyl]-3-phenylpropenoic acid (Compound 51)

In 3 mL of pyridine was dissolved 267 mg of ethyl Z-3-(3-aminophenyl)-3-phenylpropenoate, followed by the addition of 341 mg of 4-methoxybenzoyl chloride. After stirring for 17 hours, the reaction mixture was poured into 5 mL of 5% hydrochloric acid and 15 mL of ethyl acetate. The organic layer was obtained by separation, washed with water, dried over anhydrous magnesium sulfate and distilled under reduced pressure to remove the solvent. The residue was dissolved in 6 mL of methanol. To the resulting solution was added 4 mL of 5% sodium hydroxide and the mixture was stirred under heat at 70°C. Two hours later, methanol was distilled off under reduced pressure. The residue was adjusted to pH 1 with 5% hydrochloric acid and extracted three times, each with 15 mL of ethyl acetate. The organic layers were combined, washed with water, dried over anhydrous magnesium sulfate and distilled under reduced pressure to remove the solvent. The solid thus precipitated was dispersed in diisopropyl ether and then, collected by filtration, whereby 230 mg of the title compound was obtained as colorless powder.
¹H-NMR (DMSO-d₆) δ:
3.83(3H,s), 6.38(1H,s), 6.91(1H,d,J=7Hz), 7.02-7.06(3H,m), 7.31-7.40(4H,m), 7.59(1H,s), 7.85-7.96(4H,m), 10.11(1H,brs), 12.32(1H,brs)

### Example 4 (2)

In a similar manner to Example 4 (1), the following Compound (52) was synthesized.

### Z-3-[4-(4-methoxybenzenesulfonylamino)phenyl]-3-phenylpropenoic acid (Compound 52)

¹H-NMR (DMSO-d₆) δ:
3.80(3H,s), 6.25(1H,s), 6.99-7.18(8H,m), 7.33-7.37(3H,m), 7.70-7.7,3(2H,m), l0..26(1H,brs), 12.08(1H,brs

### Examples 5 (1) to 5 (2)

In a simliar manner to Example 3 (1), the following Compounds 53 and 54 were synthesized.

### Example 5 (1)

### Z-3-[3-(4-Methoxybenzoylamino)phenyl]-3-phenylpropenohydroxamic acid (Compound 53)

Appearance: Colorless crystalline powder
Melting point: 134 to 137°C
¹H-NMR (DMSO-d₆) δ:
3.84(3H,s), 6.29(1H,s) 6.82-6.90(11H,m), 7.95(2H,d,J=8Hz), 8.82(1H,brs), 1007(1H,brs), 10.59(1H,brs)

### Example 5 (2)

### Z-3-[4-(4-Nethoxybenzenesulfonylamino)phenyl]-3-phenylpropenohydroxamic acid (Compound 54)

Appearance: Colorless crystalline powder
Melting point: 188 to 191°C
¹H-NMR (DMSO-d₆) δ:
3.81(3H,s), 6.17(1H,s), 6.98-7.13(8H,m), 7.34-7.73(3H,m), 7.73(2H,d,J=9Hz), 8.79(1H,brs), 10.25(1H,brs), 10.54 (1H,brs)

### Example 6 (1)

### Synthesis of Z-3-[3-[3-(4-methoxyphenyl)ureido]phenyl]-3-phenylpropenoic acid (Compound 55)

In 3 mL of chloroform was dissolved 133 mg of ethyl Z-3-(3-aminophenyl)-3-phenylpropenoate, followed by the addition of 75 mg of 4-methoxyphenyl isocyanate. After stirring for 2 hours, the reaction mixture was poured into 15 mL of ethyl acetate and 5 mL of water. The organic layer was obtained by separation, washed with water, dried over anhydrous magnesium sulfate and distilled under reduced pressure to remove the solvent. The residue in the solid form was dissolved in 6 mL of methanol. To the resulting solution was added 4 mL of 5% sodium hydroxide, followed by stirring under heat at 70°C. Two hours later, methanol was distilled off under reduced pressure. The residue was adjusted to pH 1 with 5% hydrochloric acid and then extracted three times, each with 15 mL of ethyl acetate. The organic layers were combined, washed with water, dried over anhydrous magnesium sulfate and distilled under reduced pressure to remove the solvent. The solid thus precipitated was dispersed in diisopropyl ether and then collected by filtration, whereby 193 mg of the title compound was obtained as a colorless solid.
¹H-NMR (DMSO-d₆) δ:
3.71(3H,s), 6.35(1H,s), 6.75(1H,d,J=7Hz) 6.85(2H,d,J=9Hz), 7.26-7.42(10H,m) , 8.41(1H,brs 8.65(1H,brs), 12.16(1H,brs)

### Example 6 (2)

In a similar manner to Example 6 (1), the following Compound 56 was synthesized.

### Z-3- [3-[3-(4-Methoxyphenyl)thioureido]phenyl]-3-phenylpropenoic acid (Compound 56)

¹H-NMR (DMSO-d₆) δ:
3.75(3H,s), 6.35(1H,s), 6.89-6.91(3H,m), 7.25-7.39(9H,m), 7.60(1H,d,J=9Hz), 9.56(1H,brs), 9.70(1H,brs), 12.20(1H,brs)

### Examples 7 (1) to (2)

In a similar manner to Example 3 (1), the following Compounds 57 and 58 were synthesized.

### Example 7 (1)

### Z-3-[3-[3-(4-Methoxyphenyl)ureido]phenyl]-3-phenylpropenohydroxamic acid (Compound 57)

Appearance: Colorless crystalline powder
Melting point: 190 to 192°C
¹H-NMR (DMSO-d₆) δ:
3.72(3H,s), 6.28(1H,s), 6.78(1H,d,J=8Hz), 6.86(2H,d,J=9Hz), 7.21-7.44(10H,m), 8.39(1H,s), 8.61(1H,s 8.83(1H,brs), 10.58 (1H,brs)

### Example 7 (2)

### Z-3-[3-[3-(4-Methoxyphenyl)thioureido]phenyl]-3-phenylpropenohydroxamic acid (Compound 58)

Appearance: Colorless crystalline powder
Melting point: 176 to 179°C
¹H-NMR (DMSO-d₆) δ: 3.74(3H,s), 6.27(1H,s), 6.87-6.89(3H,m), 7.22-7.54(10H,m), 8.85(1H,brs), 9.52(1H,brs), 9.72(1H,brs), 10.55(1H,brs)

### Example 8 (1)

### Synthesis of Z-3-[3-[N-(4-methoxybenzenesulfonyl)-N-methylamino]phenyl]-3-phenylpropenoic acid (Compound 59)

In 5 mL of N,N-dimethylformamide was dissolved 210 mg of ethyl 2-3-[3-(4-methoxybenzenesulfonylamino)phenyl]-3-phenylpropenoate, followed by the addition of 27 mg of sodium hydride (60% in oil). After evolution of a hydrogen gas stopped, 100 µl of methyl iodide was added. After 21 hours and 30 minutes later, 10 mL of water and 15 mL of ethyl acetate were added. The organic layer was obtained by separation, and the water layer was extracted three times, each with 15 mL of ethyl acetate. All the organic layers were combined, washed with water and dried over anhydrous magnesium sulfate and distilled under reduced pressure to remove the solvent. The residue was dissolved in a mixture of 2 mL of methanol and 2 mL of tetrahydrofuran. To the resulting solution was added 3 mL of 5% sodium hydroxide. The mixture was stirred under heat at 65°C. Three hours later, the organic solvent was distilled off under reduced pressure. The residue was adjusted to pH 1 with 5% hydrochloric acid and extracted three times, each with 15 mL of ethyl acetate. All the organic layers were combined, washed with water, dried over anhydrous magnesium sulfate and distilled under reduced pressure to remove the solvent, whereby 0.14 g of the title compound was obtained as a solid.
¹H-NMR (DMSO-d₆) δ:
3.07(3H,s), 3.80(3H,s), 6.37(1H,s), 6.77(1H,s), 7.02(2H,d,J=9Hz), 7.06(2H,d,J=8Hz), 7.18-7.23(4H,m), 7.36-7.43(5H,m), 12.23(1H,brs)

### Examples 8 (2) and (3)

In a similar manner to Example 8 (1), the following Compounds 60 and 61 were synthesized.

### Example 8 (2)

### Z-3-[3-[N- (4-Methoxybenzenesulfonyl)-N-isopropylamino]phenyl]-3-phenylpropenoic acid (Compound 60)

¹H-NNR (DMSO-d₆) δ:
0.97(6H,d,J=7Hz), 3.79(3H,s), 4.47(1H,septet,J=7Hz), 6.37(1H,s) 6.22(1H, 6.99(2H,d,J=9Hz), 7.12-7.21(4H,m), 7.40-7.44(4H,m), 7.61(2H,d,J=9Hz), 12.26(1H,brs)

### Example 8 (3)

### Z-3-[3-[N-(2-Nitrobenzenesulfonyl)-N-methylamino]phenyl]-3-phenylpropenoic acid (Compound 61)

¹H-NMR (DMSO-d₆) δ:
3.28(3H,s), 6.38(1H,s), 6.97(1H,s), 7.11(1H,d,J=8Hz), 7.22-7.40(7H,m), 7.57-7.59(1H,m), 7.69(1H,t,J=8Hz), 7.85-7.88(1H,m), 7.96(1H,t,J=8Hz), 12.25(1H,brs)

### Examples 9 (1) to 9 (17)

In a similar manner to Example 3 (1), the following Compound 62 to Compound 76 were synthesized.

### Example 9 (1)

### Z-3-[3-[N-(4-Methoxybenzenesulfonyl)-N-methylamino]phenyl]-3-phenylpropenohydroxamic acid (Compound 62)

Appearance: Colorless powder
¹H-NMR (DMSO-d₆) δ:
3.07(3H,s), 3.80(3H,s), 6.30(1H,s), 6.74(1H,s), 7.01-7.44(12H,m), 8.85(1H,brs), 10.60(1H,brs)

### Example 9 (2)

### Z-3-[3-[N-(4-Methoxybenzenesulfonyl)-N-isopropylamino] phenyl]-3-phenylpropenohydroxamic acid (Compound 63)

Appearance: Colorless powder
¹H-NMR (DMSO-d₆) δ:
0.95(6H,d,J=6Hz), 3.79(3H,s), 4.43(1H,m), 6.29(1H,s), 6.61(1H,s), 6.97(2H,d,J=8Hz), 7.09-7.39(8H,m), 7.60(2H,d,J=8Hz), 8.83(1H,brs), 10.62(1H,brs)

### Example 9 (3)

### Z-3-[3-[N-(4-Methoxybenzenesulfonyl)-N-ethylamino]phenyl]-3-phenylpropenohydroxamic acid (Compound 64)

Appearance: Brown powder
¹H-NMR (DMSO-d₆) δ:
1.00(3H,t,J=8Hz), 3.50(2H,q,J=8Hz), 3.78(3H,s), 6.30(1H,s), 6.61(1H,s) 6.99(2H,d,J=7Hz); 7.08-7.39(8H,m), 7.60(2H,d,J=8Hz), 8.85(1H,brs), 10.60(1H,brs)

### Example 9 (4)

### Z-3-[3-[N-(2-Naphthalenesulfonyl)-N-benzylamino]phenyl]-3-phenylpropenohydroxamic acid (Compound 65)

Appearance: Pale brown powder
Melting point: 88 to 90°C
¹H-NMR (DMSO-d₆) δ:
4.80(2H,s), 6.23(1H,s), 6.77(1H,s), 7.07-8.27(20H,m), 8.88(1H,brs), 10.59(1H,brs)

### Example 9 (5)

### Z-3-[3-[N-(2-Naphthalenesulfonyl)-N-(2-phenylethyl)amino]phenyl]-3-phenylpropenohydroxamic acid (Compound 66)

Appearance: Colorless crystalline powder
Melting point: 127 to 129°C
¹H-NMR (DMSO-d₆) δ:
2.72(2H,m), 3.84(2H,t,J=7Hz), 6.25(1H,s), 6.71(1H,s 6.94-7.32(13H,m), 7.65-7.74(3H,m), 8.02-8.17(3H,m), 8.27(1H,s), 8.84(1H,brs), 10.59(1H,brs)

### Example 9 (6)

### Z-3-[3-[N-(2-Naphthalenesulfonyl)-N-(3-pyridylmethyl)amino]phenyl]-3-phenylpropenohydroxamic acid (Compound 67)

Appearance: Colorless crystalline powder
Melting point: 168 to 170°C
¹H-NMR (DMSO-d₆) δ:
4.84(2H,s), 6.23(1H,s), 6.78(1H,s), 6.84-7.31(9H,m), 7.67-7.74(4H,m), 8.02-8.17(3H,m), 8.42-8.43(3H,m), 8.95(1H,brs), 10.60(1H,brs)

### Example 9 (7)

### Z-3-[3-[N-(4-Nitrobenzenesulfonyl)-N-methylamino]phenyl]-3-phenylpropenohydroxamic acid (Compound 68)

Appearance: Colorless crystalline powder
Melting point: 153 to 155°C
¹H-NMR (DMSO-d₆) δ:
3.16(3H,s), 6.29(1H,s), 6.77(1H,s), 7.08-7.37(8H,m), 7.90(2H,d,J=9Hz), 8.33(2H,d,J=9Hz), 8.85(1H,brs), 10.60(1H,brs)

### Example 9 (8)

### Z-3-[3-[N-(2-Nitrobenzenesulfonyl)-N-methylamino]phenyl]-3-phenylpropenohydroxamic acid (Compound 69)

Appearance: Pale brown powder
¹H-NMR (DMSO-d₆) δ:
3.28(3H,s), 6.33(1H,s), 6.89(1H,s), 7.10-7.40(8H,m), 7.87-7.95(2H,m), 8.26(1H,s), 8.55(1H,m), 8.86(1H,brs), 10.62(1H,brs)

### Example 9 (9)

### Z-3-[3-[N- (3-Nitrobenzenesulfonyl)-N-methylamino]phenyl]-3-phenylpropenohydroxamic acid (Compound 70)

Appearance: Colorless powder
¹H-NMR (DMSO-d₆) δ:
3.22(3H,s), 6.31(1H,s), 6.95(1H,s 7.09(1H,d,J=8Hz), 7.16-7.17(2H,m), 7.26(1H,d,J=8Hz), 7.36-7.39(4H,m), 7.64(1H,d,J=9Hz) 7.71-7.75(1H,m), 7.86(1H,t,J=8Hz), 7.95(1H, d, J=8Hz), 8.87(1H,brs), 10.63(1H,brs)

### Example 9 (10)

### Z-3-[3-[N-(4-Aminobenzenesulfonyl)-N-methylamino]phenyl]-3-phenylpropenohydroxamic acid (Compound 71)

Appearance: Colorless powder
¹H-NMR (DMSO-d₆) δ:
3.02(3H,s), 6.03(2H,brs), 6.28(1H,s 6.52(2H,d,J=9Hz), 6.82(1H,s 7.00(1H,d,J=7Hz), 7.10-7.15(5H,m), 7.29(1H,t,J=8Hz), 7.38-7.39(3H,m), 8.81(1H,brs), 10.55 (1H,brs)

### Example 9 (11)

### Z-3- [3- [N- (2-Naphthalenesulfonyl) -N-methylamino]phenyl] -3-phenylpropenohydroxamic acid (Compound 72)

Appearance: Pale red powder
¹H-NMR (DMSO-d₆) δ:
3.19(3H,s), 6.27(1H,s), 6.84(1H,s), 7.04-7.35(9H,m), 7.48(1H,d,J=8Rz), 7.66-7.74(2H,m), 8.14(1H,dd,J=5Hz,9Hz), 8.17(1H,d,J=9Hz), 8.29(1H,m), 8.86(1H,s), 10.60(1H,brs)

### Example 9 (12)

### Z-3-[3-[N-(8-Quinolinesulfonyl)-N-methylamino]phenyl]-3-phenylpropenohydroxamic acid (Compound 73)

Appearance: Pale yellow powder
¹H-NMR (DMSO-d₆) δ:
3.63(3H,s), 6.19(1H,s), 6.61(1H,s), 6.81-7.36(8H,m), 7.64-7.68(2H,m), 8.19(1H,d,J=8Hz), 8.27(1H,d,J=9Hz), 8.49(1H,d,J=9Hz), 8.81(1H,s 9.05(1H,s), 10.54(1H,brs)

### Example 9 (13)

### Z-3-[3-[N-(5-(Dimethylamino)-1-naphthalenesulfonyl)-N-methylamino] phenyl] -3-phenylpropenohydroxamic acid (Compound 74)

Appearance: Pale yellow crystalline powder
Melting point: 96 to 99°C
¹H-NMR (DMSO-d₆) δ:
2.79(6H,s), 3.20(3H,s), 6.24(1H,s), 6.83(1H,s) 6.98-7.02(3H,m), 7.17-7.41(7H,m), 7.60(1H,t,J=9Hz), 7.87(1H,d,J=9Hz) 8.05(1H,d,J=7Hz), 8.47(1H,d,J=9Hz), 8.81(1H,brs), 10.57(1H,brs)

### Example 9 (14)

### Z-3- [3-[N- (3, 4-Dimethoxybenzenesulfonyl)-N-methylamino]phenyl]-3-phenylpropenohydroxamic acid (Compound 75)

Appearance: Colorless crystalline powder
Melting point: 98 to 101°C
¹H-NMR (DMSO-d₆) δ:
3.09(3H,s), 3.62(3H,s), 3.80(3H,s), 6.30(1H,s) 6.56(2H,m), 7.05-7.38(10H,m), 8.84(1H,brs), 10.61(1H,brs)

### Example 9 (15)

### Z-3-[3-[N-(2,5-Dimethoxybenzenesulfonyl)-N-methylamino]phenyl]-3-phenylpropenohydroxamic acid (Compound 76)

Appearance: Colorless crystalline powder
Melting point: 151 to 153°C
¹H-NMR (DMSO-d₆) δ:
3.30(3H,s), 3.66(3H,s), 3.72(3H,s), 6.26(1H,s), 6.93-7.23(12H,m), 8.83(1H,brs), 10.60(1H,brs)

### Example 9 (16)

### 3,3-bis[3-[N-(4-Methoxybenzenesulfonyl)-N-methylamino]phenyl]propenohydroxamic acid (Compound 77)

Appearance: Colorless powder
¹H-NMR (DMSO-d₆) δ:
3.06(3H,s), 3.08(3H,s), 3.81(3H,s), 3.85(3H,s), 6.26(1H,s), 6.81(1H,brs , 6.96-7.48(15H,m), 8.89(1H,brs), 10.63(1H,brs)

### Example 9 (17)

### 3,3-bis[3-[N-(4-Methoxybenzenesulfonyl)-N-ethylamino]phenyl]propenohydroxamic acid (Compound 78)

Appearance: Colorless powder
¹H-NMR (DMSO-d₆) δ:
0.90-0.96(6H,m), 3.46-3.49(4H,m), 3.79(3H,s), 3.84(3H,s), 6.26(1H,s), 6.63(1H,m), 6.82(1H,m), 7.00-7.48(14H,m), 8.88(1H,brs), 10.62(1H,brs)

### Referential Example 3

### 2-(4-Methoxybenzenesulfonylamino)-5-nitrobenzophenone (Compound 79)

In a 4 mL pyridine solution of 510 mg of 2-amino-5-nitrobenzophenone was added 520 mg of 4-methoxybenzenesulfonyl chloride, followed by stirring. After 15 hours, the reaction mixture was poured into 10 mL of 5% hydrochloric acid and 20 mL of ethyl acetate. The organic layer was obtained by separation, washed with water, dried over anhydrous magnesium sulfate and distilled under reduced pressure to remove the solvent. The residue was subjected to chromatography on a silica gel column (25cc, chloroform), whereby 150 mg of the title compound was obtained as a yellow oil.

### Example 10

### Synthesis of ethyl E-3-[2-(4-methoxybenzenesulfonylamino)-5-nitrophenyl]-3-phenylpropenoate (Compound 80)

In a suspension of 90 mg (60% in oil) of sodium hydride in tetrahydrofuran, was added dropwise 2 mL of a tetrahydrofuran solution of 430 mg of triethyl phosphonoacetate under ice cooling. To the reaction mixture was added 140 mg of 2-(4-methoxybenzenesulfonylamino) -5-nitrobenzophenone, followed by stirring under heat at 60 to 70°C. After 23 hours, tetrahydrofuran was distilled off under reduced pressure. Water and ethyl acetate were then added to the residue. The organic-layer was obtained by separation, dried over anhydrous magnesium sulfate and distilled under reduced pressure to remove the solvent. The residue was subjected to chromatography on a silica gel column (200 cc, hexane:ethyl acetate = 5:1), whereby 80 mg of the title compound was obtained as colorless crystals. No Z-isomer was obtained.
Melting point: 126 to 129°C
¹H-NMR (CDCl₃) δ:
1.19(3H,t,J=7Hz), 3.84(3H,s), 4.14(2H,q,J=7Hz), 5.88(1H,s), 6.82-6.89(4H,m), 7.12-7.70(6H,m), 8.07(1H,d,J=3hz), 8.17(1H,dd,J=3Hz,7Hz)

### Example 11

### Synthesis of E-3-[2-(4-methoxybenzenesulfonylamino)-5-nitrophenyl]-3-phenylpropenohydroxamic acid (Compound 81)

In 2 mL of methanol was dissolved 160 mg of ethyl E-3-[2-(4-methoxybenzenesulfonylamino)-5-nitrophenyl]-3-phenylpropenoate. To the resulting solution was added 2 mL of 5% sodium hydroxide, followed by stirring under heat at 50 to 60°C. Eight hours later, methanol was distilled off under reduced pressure. The residue was adjusted to pH 1 with 5% hydrochloric acid and extracted thee times, each with 15 mL of ethyl acetate. The organic layers were combined, washed with water, dried over anhydrous magnesium sulfate and distilled under reduced pressure to remove the solvent. The residue was dissolved in 3 mL of N,N-dimethylformamide, followed by the successive addition of 80 mg of hydroxybenzotriazole, 70 mg of 1-ethyl-3-(3-dimethylaminopropyl)carbodiimide hydrochloride, 60 mg of N-methylmorpholine and 90 mg of O-(tert-butyldimethylsilyl)hydroxylamine. After stirring for 20 hours and 30 minutes, the reaction mixture was diluted with 10 mL of ethyl acetate, washed with water, dried over anhydrous magnesium sulfate and distilled under reduced pressure to remove the solvent. The residue in the oil form was subjected to chromatography on a silica gel column (10 cc, chloroform → chloroform:methanol = 30:1), whereby 14 mg of the title compound was obtained as colorless crystalline powder.
Appearance: Colorless crystalline powder
Melting point: 182 to 185°C
¹H-NMR (DMSO-d₆, δ):
3.83(3H,s), 5.67(1H,s), 7.04-7.60(10H,m), 7.82(1H,d,J=3Hz), 8.15(1H,m), 8.99(1H,brs) 9.84(1H,brs), 10.54(1H,brs)

### Referential Example 4

### Synthesis of 3-(3-nitrobenzoyl)pyridine (Compound 82)

In 52 mL of 36% sulfuric acid was dissolved 10.22 g of 3-benzoylpyridine. To the resulting solution was added 6.27 g of potassium nitrate in portions under ice cooling. At the temperature returned to room temperature, the mixture was stirred. Three hours later, the reaction mixture was poured into ice water. The mixture was neutralized with 20% sodium hydroxide and extracted with ethyl acetate. The organic layer was washed with water, dried over anhydrous magnesium sulfate and distilled under reduced pressure to remove the solvent. The crystals thus precipitated were collected by filtration and dried, whereby 9.43 g of the title compound was obtained as yellow crystals.
Melting point: 73 to 76°C

### Example 12

### Synthesis of ethyl E,Z-3-[3-(4-methoxybenzenesulfonylamino)phenyl]-3-(3-pyridyl)propenoate (Compound 83)

In 30 mL of pyridine was dissolved 14.4 g of ethyl E,Z-3-(3-aminophenyl)-3-(3-pyridyl)propenoate. To the . resulting solution was added 12.9 g of 4-methoxybenzenesulfonyl chloride, followed by stirring. After 22 hours, the reaction mixture was poured into 50 mL of 5% hydrochloric acid and 300 mL of ethyl acetate. The organic layer was obtained by separation, washed with water, dried over anhydrous magnesium sulfate and distilled under reduced pressure to remove the solvent. Vacuum drying of the residue yielded 24.1 g of a mixture of the title compound as a yellow oil.

### Example 13

### Synthesis of ethyl E,Z-3-[3-(4-methoxybenzenesulfonylamino)phenyl]-3-(3-pyridyl-N-oxide)propenoate (Compound 84)

In 50 mL of dichloromethane was dissolved 6.80 g of ethyl E,Z-3-[3-(4-methoxybenzenesulfonylamino)phenyl]-3-(3-pyridyl)propenoate. To the resulting solution was added 10.8 g of m-chloroperbenzoic acid, followed by stirring. After 19 hours, the reaction mixture was diluted with 100 mL of dichloromethane and washed successively with 10% sodium thiosulfate, saturated sodium bicarbonate and water. The organic layer was dried over anhydrous magnesium sulfate and distilled under reduced pressure to remove the solvent, whereby 6.19 g of a mixture of the title compound was obtained as a yellow oil.
¹H-NMR (CDCl₃, δ):
1.09(3H,t,J=7Hz), 1.20(2H,t,J=7Hz), 3.82(2H,s), 3.83(3H,s), 4.03(2H,t,J=7Hz), 4.06(4/3H,t,J=7Hz), 6.38(1H,s), 6.40(2/3H,s), 6.88-7.34(13H+2/3H,m) 7.67-7.71(3H,m), 8.00(1H,s), 8.03(2/3H,s), 8.21-8.23(1H,m), 8.27(2/3H,d,J=8Hz).

### Example 14

### Synthesis of ethyl E,Z-3-[3-[N-(4-methoxybenzenesulfonyl)-N-isopropylamino]phenyl]-3-(3-pyridyl N-oxide)propenoate (Compound 85)

In 4 mL of N,N-dimethylformamide was dissolved 0:92 g of E,Z-3-[3-(4-methoxybenzenesulfoneamido)phenyl]-3-(3-pyridyl N-oxide)propenoate. To the resulting solution was added 0.09 g (60% in oil) of sodium hydride. After evolution of a hydrogen gas stopped, 600 µL of isopropyl iodide was added. The mixture was stirred for 16 hours and 30 minutes. The reaction mixture was then diluted with 30 mL of ethyl acetate and washed twice each with 5 mL of water. The organic layer was dried over anhydrous magnesium sulfate and distilled under reduced pressure to remove the solvent. The residue in the oil form was subjected to chromatography on a silica gel column (30 cc, chloroform → chloroform:methanol = 100:1), whereby 0.35 g of a mixture of the title compound was obtained as a pale yellow oil.
¹H-NMR (CDCl₃, δ):
1.02(3H,d,J=7Hz), 1.07(6H,d,J=7Hz), 1.15(3H,t,J=7Hz), 1.21(1.5H,t,J=7Hz), 3.85(3H,s), 3.90(1.5H,s), 4.07(2H,q,J=8Hz), 4.12(1H,q,J=8Hz), 4.57-4.62(1.5H,m), 6.375(1H,s), 6.381(0.5H,s), 6.88-6.96(4H,m), 7.07-7.39-(8H,m), 7.62-7.66(3H,m), 8.05(0.5H,s), 8.11(1H,s), 8.19(1H,d,J=8Hz), 8.22(0.5H,d,J=8Hz).

### Example 15

### Synthesis of ethyl E,Z-3-[3-[N-(4-methoxybenzenesulfonyl)-N-isopropylamino]phenyl]-3- (3-pyridyl)propenoate (Compound 86)

In 15 mL of acetic acid was dissolved 1.76 g of ethyl E,Z-3-[3-[N-(4-methoxybenzenesulfonyl)-N-isopropylamino]phenyl]-3- (3-pyridyl N-oxide)propenoate. To the resulting solution was added 0.71 g of iron powder and the mixture was stirred at 70 to 80°C. One hour later, the temperature was returned to room temperature. The reaction mixture was neutralized with saturated sodium bicarbonate. The insoluble matters thus precipitated were filtered off. The filtrate was extracted with ethyl acetate. The organic layer was washed with water, dried over anhydrous magnesium sulfate and distilled under reduced pressure to remove the solvent. The residue in the oil form was subjected to chromatography on a silica gel column (100 cc, chloroform → chloroform:methanol = 100:1), whereby 1.39 g of a mixture of the title compound was obtained as a pale yellow oil.

### Example 16

In a similar manner to Example 2 (1) except that the title compound was subjected further to chromatography on a silica gel column (600 cc, chloroform → chloroform:methanol =40:1), whereby the following Compound 87 was obtained.

### E-3-[3-[N-(4-Methoxybenzenesulfonyl)-N-isopropylamino]phenyl]-3-(3-pyridyl)propenoic acid (Compound 87)

¹H-NMR (DMSO-d₆) δ:
0.96(6H,d,J=7Hz), 3.80(3H,s), 4.46(1H,septet,J=7Hz), 6.49(1H,s), 6.66(1H,s), 6.66-7.03(2H,m), 7.13-7.19(2H,m), 7.42-7.54(3H,m), 7.57-7.62(2H,m), 8.58(1H,d,J=2Hz), 8.59(1H,d,J=2Hz), 12.42(1H,brs)

### Examples 17 (1) to (5)

In a similar manner to Example 3 (1), the following Compounds 88 to 92 were synthesized.

### Example 17 (1)

### E-3-[3-[N-(4-Methoxybenzenesulfonyl) -N-isopropylamino]phenyl]-3-(3-pyridyl)propenohydroxamic acid (Compound 88)

Appearance: Colorless crystalline powder
Melting point: 158 to 160°C
¹H-NMR (DMSO-d₆) δ:
0.96(6H,d,J=7Hz), 3.80(3H,s), 4.43(1H,septet,J=6Hz), 6.38(1H,s), 6.65(1H,s), 6.98(2H,d,J=9Hz), 7.11(1H, d, J=3Hz), 7.20(1H,d,J=3Hz), 7.39-7.50(3H,m), 7.61(2H,d,J=8Hz), 8.39(1H,s), 8.58(1H,d,J=4Hz), 8.90(1H,brs) 10.67(1H,brs)

### Example 17 (2)

### E-3-[3-[N-(4-Methoxybenzenesulfonyl)-N-methylamino]phenyl]-3-(3-pyridyl)propenohydroxamic acid (Compound 89)

Appearance: Colorless powder
¹H-NMR (DMSO-d₆) δ:
3.06(3H,s), 3.81(3H,s), 6.38(1H,s), 6.78(1H,s), 7.02(2H,d,J=9Hz), 7.08-7.50(7H,m), 8.39(1H,s), 8.56(1H,d,J=4Hz), 8.92(1H,brs), 10.65(1H,brs)

### Example 17 (3)

### E-3-[3-[N-(4-Methoxybenzenesulfonyl)-N-butylamino]phenyl]-3-(3-pyridyl)propenohydroxamic acid (Compound 90)

Appearance: Colorless powder
¹H-NMR (DMSO-d₆) δ:
0.86(3H,t,J=8Hz), 1.24-1.32(4H,m), 3.45(2H,t,J=8Hz), 3.79(3H,s), 6.38(1H,s), 6.65(1H,s), 7.00(2H,d,J=8Hz), 7.12-7.17(2H,m), 7.37-7.49(5H,m), 8.38(1H,s), 8.57(1H,dd,J=2Hz,5Hz), 8.90(1H,brs), 10.69(1H,brs)

### Example 17 (4)

### E-3-[3-[N-(4-Methoxybenzenesulfonyl)-N-isobutylamino]phenyl]-3-(3-pyridyl)propenohydroxamic acid (Compound 91)

Appearance: Colorless powder
¹H-NMR (DMSO-d₆) δ:
0.86(3H,d,J=8Hz), 1.51-1.54(1H,m), 3.24(2H,d,J=8Hz), 3.79(3H,s), 6.38(1H,s), 6.68(1H,s), 7.00(2H,d,J=9Hz), 7.12(1H,d,J=8Hz), 7.18(1H,d,J=8Hz,), 7.36-7.50(5H,m), 8.39(1H, 8.58(1H,dd,J=3Hz) 8.89(1H,brs), 10.64(1H,brs)

### Example 17 (5)

### E-3-[3-[N-(3,4-Dimethoxybenzenesulfonyl)-N-methylamino]phenyl]-3-(3-pyridyl)propenohydroxamic acid (Compound 92)

Appearance: colorless powder
¹H-NMR (DMSO-d₆) δ:
3.09(3H,s), 3.67(3H,s), 3.81(3H,s), 6.39(1H,s), 6.87-6.89(2H,m), 7.06-7.49(7H,m), 8.41(1H,s), 8.56(1H,d,J=3Hz), 8.90(1H,brs), 10.66(1H,brs)

### Example 18 (1)

### Synthesis of E-3-[3-[N-(4-methoxybenzenesulfonyl)-N-isopropylamino]phenyl]-3-(3-pyridyl N-oxide)propenohydroxamic acid (Compound 93)

In 3 mL of dioxane was dissolved 270 mg of ethyl E,Z-3-[3-[N-(4-methoxybenzenesulfonyl)-N-isopropylamino]phenyl]-3-(3-pyridyl N-oxide)propenoate. To the resulting solution was added 2 mL of 5% sodium hydroxide and the mixture was stirred at room temperature. After one hour and 40 minutes, dioxane was distilled off under reduced pressure. The residue was diluted with water The mixture was adjusted to pH 5 to 6 with 5% hydrochloric acid and then, extracted with ethyl acetate. The organic layer was washed with water, dried over anhydrous magnesium sulfate and distilled under reduced pressure to remove the solvent. Without purifying 0.19 g of the residue in the oil form, it was dissolved in 1.5 mL of N,N-dimethylformamide, followed by successive addition of 76 mg of hydroxybenzotriazole, 114 mg of 1-ethyl-3-(3-dimethylaminopropyi)carbodiimide hydrochloride, 50 mg of N-methylmorpholine and 123 mg of o-(tert-butyldimethylsilyl)hydroxylamine. After stirring for 22 hours, 5 mL of water was added to the reaction mixture. The mixture was extracted three times, each with 15 mL of a chloroform-tetrahydrofuran mixture (4:1). All the organic layers were combined, dried over anhydrous magnesium sulfate and distilled under reduced pressure to remove the solvent. The residue was subjected to chromatography on a silica gel column (15 cc, chloroform → chloroform:methanol = 25:1), whereby 34 mg of the title compound was obtained as colorless powder.
Appearance: colorless powder
¹H-NMR (DMSO-d₆) δ:
0.97(6H,d,J=7Hz), 3.82(3H,s), 4.45(1H,septet,J=6Hz), 6.45(1H,s), 6.66(1H,s), 7.04(3H,d,J=8Hz), 7.13(1H,d,J=8Hz), 7.23(1H,d,J=8Hz), 7.44(2H,t,J=8Hz), 7.59-7.64(2H,m), 7.94(1H,s), 8.26(1H,d,J=7Hz), 8.95(1H,brs), 10.68(1H,brs)

### Examples 18 (2) to (9)

In a similar manner to 18 (1), the following Compounds 94 to 101 were synthesized.

### Example 18 (2)

### E-3-[3-[N-(4-Methoxybenzenesulfonyl)-N-methylamino]phenyl]-3-(3-pyridyl N-oxide)propenohydroxamic acid (Compound 94) Appearance: Colorless powder

¹H-NMR (DMSO-d₆) δ:
3.07(3H,s), 3.80(3H,s), 6.46(1H,s), 6.80(1H,s), 7.04-7.23(5H,m), 7.36-7.44(4H,m), 7.92(1H,s), 8.24(1H,d,J=6Hz), 8.97(1H,brs), 10.66(1H,brs)

### Example 18 (3)

### E-3-[3-[N-(3,4-Dimethoxybenzenesulfonyl)-N-methylamino]phenyl]-3-(3-pyridyl N-oxide)propenohydroxamic acid (Compound 95)

Appearance: Colorless powder
¹H-NMR (DMSO-d₆) δ:
3.08(3H,s), 3.67(3H,s), 3.81(3H,s), 6.47(1H,s), 6.89-7.43(9H,m), 7.95(1H,s), 8.21-8.23(1H,m), 8.94(1H,brs) 10. 66(1H,brs)

### Example 18 (4)

### E-3-[3-[N-(3,4-Dimethoxybenzenesulfonyl)-N-methylamino]phenyl]-3- (4-pyridyl)propenohydroxamic acid (Compound 96)

Appearance: Colorless powder
¹H-NMR (DMSO-d₆) δ:
3.05(3H,s), 3.65(3H,s), 3.86(3H,s), 6.39(1H,s), 6.77(1H,s), 6.80(1H,m), 7.05-7.21(6H,m), 7.38(1H,t,J=8Hz), 8.53(2H,d,J=6Hz), 8.94(1H,brs), 10. 74 (1H, brs)

### Example 18 (5)

### E-3-[3-[N-(3,4-Dimethoxybenzenesulfonyl)-N-methylamino]phenyl]-3-(4-pyridyl N-oxide)propenohydroxamic acid (Compound 97)

Appearance: Colorless powder
¹H-NMR (DMSO-d₆) δ:
3.07(3H,s), 3.68(3H,s), 3.86(3H,s), 6.31(1H,s), 6.81(1H,m), 6.89(1H,m), 6.91-7.41(7H,m), 8.17(2H,d,J=7Hz), 8.94(1H,brs), 10.66(1H,brs)

### Example 18 (6)

### E-3-[3-[N-(4-Methoxybenzenesulfonyl)-N-methylamino]phenyl]-3-(4-pyridyl N-oxide)propenohydroxamic acid (Compound 98) Appearance: Colorless powder

¹H-NMR (DMSO-d₆) δ:
3.07(3H,s), 3.81(3H,s), 6.49(1H,s) 6.74(1H,m), 7.04-7.45(8H,m), 8.21-8.32(3H,m), 9.02(1H,brs), 10.78(1H,brs)

### Example 18 (7)

### Z-3-[3-[N-(4-Methoxybenzenesulfonyl)-N-methylamino]phenyl]-3-(4-pyridyl N-oxide)propenohydroxamic acid (Compound 99) Appearance: Colorless powder

¹H-NMR (DMSO-d₆) δ:
3.07(3H,s), 3.86(3H,s), 6.30(1H,s), 6.90(1H,s), 7.09-7.42(9H,m), 8.17-8.18(2H,m), 9.02(1H,brs), 10.79(1H,brs)

### Example 18 (8)

### E-3-[3-[N-(4-Methoxybenzenesulfonyl)-N-methylamino]phenyl]-3-(2-pyridyl N-oxide)propenohydroxamic acid (Compound 100)

Appearance: Colorless powder
¹H-NMR (DMSO-d₆) δ:
3.04(3H,s), 3.86(3H,s), 6.52(1H,s), 6.87(1H,s), 7.04(2H,d,J=9Hz), 7.08(1H,d,J=9Hz), 7.16(1H,d,J=8Hz), 7.27(1H,t,J=8Hz), 7.39-7.44(5H,m), 8.23(1H,d,J=6Hz), 8.99(1H,brs), 10.76(1H,brs)

### Example 18 (9)

### E-3-[3-[N-(3,4-Dimethoxybenzenesulfonyl)-N-methylamino]phenyl]-3-(2-pyridyl)propenohydroxamic acid (Compound 101)

Appearance: Colorless powder
¹H-NMR (DMSO-d₆) δ:
3.04(3H,s), 3.61(3H,s), 3.84(3H,s), 6.56(1H,s), 6.74-6.75(1H,m) 6.99-7.38(9H,m), 8.18-8.19(1H,m 8.97(1H,brs), 10.76(1H,brs)

### Referential Example 5

In a similar manner to Referential Example 1 (1), the following Compound 102 was synthesized.

### Ethyl E-3-(3-nitrophenyl)-3-(3-pyridyl)propenoate (Compound 102)

Appearance: Colorless crystalline powder
Melting point: 67 to 68°C
¹H-NMR (CDCl₃) δ:
1.18(3H,t,J=7Hz), 4.11(2H,q,J=7Hz), 6.52(1H,s), 7.32(1H,dd,J=6Hz,9Hz), 7.54-7.64(3H,m), 8.12(1H,s), 8.30(1H,d,J=8Hz), 8.59(1H,s), 8.65(1H,d,J=5Hz)

### Example 19

In a similar manner to Example 13 (1), the following Compound 103 was synthesized.

### Ethyl E-3-[3-(4-methoxybenzenesulfonylamino)phenyl]-3- (N-oxidepyridin-3-yl)propenoate (Compound 103)

Appearance: Colorless crystalline powder
Melting point: 153 to 155°C
¹H-NMR (CDCl₃) δ:
1.08(3H,t,J=7Hz), 3.83(3H,s), 4.03(2H,q,J=7Hz), 6.37(1H,s), 6.89-6.92(4H,m), 6.97(1H, 7.08(1H,d,J=8Hz), 7.16(1H,d,J=8Hz), 7.23-7.27(5H,m), 7.64-7.69(2H,m), 7.98(1H,s), 8.20(1H,d,J=7Hz).

### Examples 20 (1) to (15)

In a similar manner to Example 18 (1), the following Compounds 104 to 118 were synthesized.

### Example 20 (1)

### E-3-[3-(4-Methoxybenzenesulfonylamino)phenyl]-3-(3-pyridyl)propenohydroxamic acid (Compound 104)

Appearance: Colorless powder
¹H-NMR (DMSO-d₆) δ:
3.81(3H,s), 6.35(1H,s), 6.82(1H,s), 6.88(1H,d,J=7Hz), 7.00-7.05(3H,m), 7.21(1H,t,J=8Hz), 7.35(1H,m), 7.58(2H,d,J=9Hz), 7.64(2H,m), 8.30(1H,s), 8.55(1H,s) 8.98(1H,brs), 10.00(1H,brs)

### Example 20 (2)

### E-3-[3-(4-Methoxybenzenesulfonyl)aminophenyl]-3-(2-pyrazyl)propenohydroxamic acid (Compound 105)

Appearance: Colorless powder
¹H-NMR ( DMSO-d₆) δ:
3.81(3H,s), 6.90-6.92(2H,m), 6.99-7.08(4H,m), 7.25(1H,t,J=8Hz), 7. 63 (2H, d, J=8Hz) , 7.90(1H,s), 8.63(1H,s), 8.68(1H,s) 8.94(1H,brs), 10.10(1H,brs), 10.83(1H,brs)

### Example 20 (3)

### E-3-[3-[N-(4-Methoxybenzenesulfonyl)-N-isopropylamino]phenyl]-3-(2-pyridyl)propenohydroxamic acid (Compound 106)

Appearance: Colorless powder
¹H-NMR (DMSO-d₆) δ:
0.99(6H,d,J=7Hz), 3.78(3H,s), 4.47(1H,m), 6.62(1H,s), 6.80(1H,m), 6.99(2H,m), 7.04(1H,s), 7.14-7.20(2H,m), 7.39-7.43(2H,m), 7.64(2H,m), 7.80(1H,m), 8.64(1H,s), 8.88(1H,brs), 10.80(1H,brs)

### Example 20 (4)

### E-3-[3-[N-(1-Piperidinesulfonyl)-N-methylamino]phenyl]-3-(2-pyridyl)propenohydroxamic acid (Compound 107)

Appearance: Colorless powder
¹H-NMR (DMSO-d₆) δ:
1.44(6H,s), 3.09(3H,s), 3.24(4H,s), 6.95(1H,d,J=8Hz) 7.07(1H,s), 7.09-7.10(1H,m), 7.25(1H,s), 7.39-7.42(3H,m), 7.77(1H,t,J=7Hz), 8.66(1H,brs) 8.87(1H,brs), 10.80(1H,brs)

### Example 20 (5)

### E-3-[3-[N-(4-Methoxybenzenesulfonyl)-N-isopropylamino]phenyl]-3-(4-pyridyl)propenohydroxamic acid (Compound 108)

Appearance: Colorless powder
¹H-NMR (DMSO-d₆) δ:
0.95(6H,d,J=7Hz), 3.77(3H,s), 4.43(1H,m), 6.49(1H,s), 6.60(1H,s), 6.92-7.17(6H,m), 7.40(1H,m), 7.60(2H,m), 8.57(2H,m), 8.93(1H,brs), 10.71(1H,brs)

### Example 20 (6)

### E-3-[3-[N-(3-Methoxybenzenesulfonyl)-N-isopropylamino]phenyl]-3-(4-pyridyl)propenohydroxamic acid (Compound 109)

Appearance: Colorless powder
¹H-NMR (DMSO-d₆) δ:
0.95(6H,d,J=7Hz), 3.71(3H,s), 4.46(1H,septet, J=7Hz), 6.49(1H,s), 6.66(1H,s), 7.09-7.17(6H,m), 7.25(1H,d,J=8Hz), 7.37-7.42(2H,m), 8.56(2H,d,J=5Hz), 8.88(1H,brs), 10.69 (1H,brs)

### Example 20 (7)

### E-3- [3-[N-(4-Methoxybenzenesulfonyl)-N-ethylamino]phenyl]-3-(3-pyridyl)propenohydroxamic acid (Compound 110)

Appearance: Colorless powder
¹H-NMR (DMSO-d₆) δ:
0.98(3H,t,J=7Hz), 3.50(2H,q,J=7Hz), 3.79(3H,s), 6.37(1H,s), 6.64(1H,s), 6.99(2H,d,J=9Hz), 7 .12 (1H,d,J=8Hz), 7.16(1H,d,J=9H 7.37-7.45(3H,m), 7.47(2H,d,J=9Hz), 8.38(1H,s), 8.56-8.57(1H,m), 8.90(1H,brs), 10.64(1H,brs)

### Example 20 (8)

### E-3- [3- [N-(4-Phenoxybenzenesulfonyl)-N-isopropylamino]phenyl]-3-(3-pyridyl)propenohydroxamic acid (Compound 111)

Appearance: Colorless powder
¹H-NMR (DMSO-d₆) δ:
0.98(6H,d,J=7Rz), 4.44(1H,septet,J=7Hz), 6.40(1H,s), 6.67(1H,s), 7.00(2H,d,J=9Hz), 7.08(2H,d,J=9Hz), 7.13(1H,d,J=9Hz) 7.19-7.27(2H,m), 7.38-7.51(5H,m), 7.68(2H,d,J=9Hz), 8.39(1H,m), 8.52(1H,d,J=4Rz), 8.89(1H,brs), 10.67(1H,brs)

### Example 20 (9)

### E-3-[3-[N-(4-Methoxybenzenesulfonyl)-N-(cyanomethyl)amino]phenyl]-3-(3-pyridyl)propenohydroxamic acid (Compound 112)

Appearance: Colorless powder
¹H-NMR (DMSO-d₆) δ:
3.83(3H,s), 4.82(2H,s), 6.39(1H,s), 6.84(1H,s), 7.05(2H,d,J=9Hz), 7.22-7.26(2H,m), 7 . 42-7.50(3H,m), 7.57(2H,d,J=9Hz), 8.38(1H,s), 8.57(1H,m), 8.98(1H,brs), 10.71 (1H,brs)

### Example 20 (10)

### Z-3-[3-[N-(4-Methoxybenzenesulfonyl)-N-(cyanomethyl)amino]phenyl)-3-(3-pyridyl)propenohydroxamic acid (Compound 113)

Appearance: Colorless powder
¹H-NMR ( DMSO-d₆) δ:
3.95(3H,s), 4.93(2H,s), 6.38(1H,s 6.83(1H,s), 7.11-7.63(9H,m), 8.38(1H,m), 8.58(1H,m), 8.99(1H,brs), 10.81(1H,brs)

### Example 20 (11)

### Z-3-[3-[N-(4-Methoxybenzenesulfonyl)-N-isopropylamino]phenyl]-3-(3-pyridyl)propenohydroxamic acid (Compound 114)

Appearance: Colorless crystalline powder
Melting point: 195 to 197°C
¹H-NMR (DMSO-d₆) δ:
0.87(6H,d,J=7Hz), 3.87(3H,s), 4.38(1H,septet,J=7Hz), 6.40(1H,s), 6.59(1H,s), 7.04(2H,dd,J=2Hz,7Hz), 7.08-7.11(2H,m), 7.34-7.48(4H,m), 7.56(2H,d,J=7Hz), 8.29(1H,s) 8.94(1H,brs), 10.76(1H,brs)

### Example 20 (12)

### E-3-[3-[N-(4-Methoxybenzenesulfony)-N-propylamino]phenyl]-3-(3-pyridyl)propenohydroxamic acid (Compound 115)

Appearance: Colorless crystalline powder
Melting point: 151 to 153°C
¹H-NMR (DMSO-d₆) δ:
0.82(3H,t,J=7Hz), 1.33-1.37(2H,m), 3.41(3H,t,J=7Hz), 6.37(1H,s), 6.66(1H,s), 7.00(2H,d,J=9Hz), 7.12(1H,d,J=8Hz), 7.17(1H,d,J=8Hz), 7.36-7.43(3H,m), 7.47(2H,d,J=8Hz), 8.38(1H,s), 8.57(1H,d,J=4Hz), 8.90(1H,brs), 10.64(1H,brs)

### Example 20 (13)

### E,Z-3-[3-[N-(4-Methoxybenzenecarbonyl)-N-methylamino]phenyl]-3-(3-pyridyl)propenohydroxamic acid (Compound 116)

Appearance: Colorless powder
¹H-NMR (DMSO-d₆) δ:
3.32(3/2H,s), 3.36(3/2H,s), 3.73(3/2H,s), 3.74(3/2H,s), 6.31(1/2H,s), 6.35(1/2H,s), 6.76-6.78(2H,m), 6.84-7.34(8H,m), 8.20(1/2H,d,J=2Hz), 8.31(1/2H,d,J=2Hz), 8.19-8.52(1H,m) 8.91(1H,brs 10.65(1/2H,brs), 10.67(1/2H,brs)

### Example 20 (14)

### E, Z-3- [3- [3N-(4-Methoxyphenyl)-1N-methylureido]phenyl]-3-(3-pyridyl)propenohydroxamic acid (Compound 117)

Appearance: Colorless powder
¹H-NMR (DMSO-d₆) δ:
3.22(3/2H,s), 3.24(3/2H,s), 3.69(3/2H,s), 3.70(3/2H,s), 6.40(1/2H,s), 6.44(1/2H,s), 6.77-6.82(2H,m), 6.99-7.04(1H,m), 7.18-7.74(4H,m), 8.10(1/2H,s), 8.32(1/2H,s), 8.38(1/2H,s), 8.51(1/2H,m), 8.56(1H,m), 8.92(1/2H,brs), 8.97(1/2H,brs), 10.73(1/2H,brs), 10.76(1/2H,brs)

### Example 20 (15)

### E-3-[3-[N-(4-Methoxybenzenesulfonyl)-N-allylamino]phenyl]-3-(3-pyridyl)propenohydroxamic acid (Compound 118)

Appearance: Colorless powder
¹H-NMR (DMSO-d₆) δ:
3.82(3H,s), 4.13(2H,d,J=6Hz), 5.06(1H,d,J=10Hz), 5.15(1H,d,J=16Hz), 5.67-5.72(1H,m), 6.39(1H,s), 6.71(1H,s), 7.03(2H,d,J=9Hz), 7.13(1H,d,J=8Hz), 7.18(1H,d,J=8Hz), 7.35-7.49(3H,m), 7.52(2H,d,J=9Hz), 8.38(1H,s) 8.57(1H,d,J=3Hz), 8.95(1H,brs), 10.66(1H,brs)

### Example 20 (16)

### E-3- [3- [N- (3, 4-Dimethoxybenzenesulfonyl)phenyl] -3- (3-pyridyl)propenohydroxamic acid (Compound 119)

Appearance: Colorless powder
¹H-NMR (DMSO-d₆) δ:
0.97(6H,d,J=5Hz), 3.70(3H,s), 3.80(3H,s), 4.40-4.55(1H,m) 6.39(1H,s) 6.71(1H,s), 6.98-7.12(4H,m), 7.21-7.25(2H,m), 7.40-7.49(2H,m), 8.39(1H,s), 8.56(1H,s), 9.04(1H,brs 10.67 (1H,brs)

### Test 1 (In vitro TACE inhibitory activity)

### (1) Crude extraction of TACE from THP-1 cell membrane

To THP-1 cells (cell density: 2 x 10⁶/mL) suspended in RPMI 1640 medium containing 1% FBS were added LPS (E. coli 055:B55, final concentration: 1 µg/mL), silica (0.014 µm, final concentration: 50 µg/mL) and hydroxyurea (final concenration: 2 mM), followed by cultivation at 37°C for 16 hours under 5%-CO₂ conditions. The resulting cells were then washed once with RPMI 1640 medium free of FBS. The cells were suspended in 3 times the pellet volume of Solution A (10 mM sodium phosphate (pH 7.4), 1 mM MgCl₂, 30 mM NaCl, 0.02% NaN₃, 5 µM PMSF (phenyl methyl sulfonyl fluoride)). The cells were ground by a Polytron homogenizer (5 sec × 5 times, ice cooling for 1 to 2 minutes each time). The homogenate solution was stacked over 41% sucrose-Solution A, followed by centrifugation (150,000 g × 1 hour). After centrifugation, the intermediate layer was collected and diluted with 4 volumes of Solution A, followed by centrifugation (150,000 g × 20 min). A sufficient amount of a solubilizing solution (1%-Triton X-100-A solution) was added (protein concentration: 1 mg/mL) and the mixture was stirred (4°C × 1 hour).
Stirring was followed by centrifugation (100,000 g × 30 min, 4°C). The supernatant was collected, which was provided for use as a TACE crude extract.

### (2) Measurement of TACE inhibitory activity

TACE (crude extracted protein from membrane fraction of THP-1 cells, final concentration: 10 µg/mL), each of test compounds (DMSO final concentration: 1%) shown in Table 1 and an incubate solution (50 mM Tris-HCl (pH 7.4), 5 mM CaCl₂, 0.002% NaN₃, 0.002% Brij 35) was added to a 96-well black plate. After pre-incubation at room temperature for 30 minutes, a substrate (N-methylanthranilyl-LAQAVRSK(DNP)rr-NH₂: product of PEPTIDE INSTITUTE, INC., final concentration: 20 µM) was added, followed by incubation at room temperature for at least 4 hours. The fluorescence intensity was measured using POLAR STAR (Ex: 340 nm, Em: 430 nm) and from it, TACE inhibitory activity (IC₅₀ value) was calculated. The results are shown in Table 1.

### Test 2 (Measurement of MMP-1 inhibitory activity)

MMP-1 inhibitory activity (IC₅₀ value) was measured in accordance with the above-described measuring method of TACE inhibitory activity by using MMP-1 (product of Cosmo Bio Co.) and, as a substrate, 7-methoxycoumarin-4-yl-acetyl-PLGL-[N³-(2,4-dinitrophenyl)2,3-diaminopropionyl]-AR-NH₂ (product of Peptide Institute Inc.). The fluorescence intensity was measured using POLAR STAR (Ex: 340 nm, Em: 405 nm). The results are shown together in Table 1.

**Table 1**

| Compound | TACE inhibitory activity IC₅₀ (nM) | MMP-1 inhibitory activity IC₅₀ (nM) |
|---|---|---|
| 62 | 8.6 | > 10000 |
| 63 | 8.6 | > 10000 |
| 68 | 35 | > 10000 |
| 73 | 29 | > 10000 |
| 75 | 13 | > 10000 |
| 88 | 6.4 | > 10000 |
| 89 | 7.0 | > 10000 |
| 93 | 13 | > 10000 |
| 94 | 13 | > 10000 |

### Test 3 (Measurement of inhibitory activity against secretion of TNF-α)

To THP-1 cells (5 × 10⁵ mL) suspended in RPMI 1640 medium containing 10% FBS were added LPS (100 ng/mL) and a medicament of various concentrations (DMSO final concentration: 0.1%), followed by incubation at 37°C for 4 hours under 5%-CO₂ conditions. The TNF-α in the cultured supernatant was analyzed by ELISA, whereby inhibitory activity (IC₅₀) against secretion of TNF-α was calculated. The results are shown in Table 2.

| Compound | Inhibitory activity against secretion of TNF-α IC₅₀ (µM) |
|---|---|
| 75 | 6.4 |
| 88 | 2.8 |
| 89 | 4.1 |
| 93 | 5.5 |

### Industrial Applicability

The compounds (1) or salts thereof according to the present invention have excellent TACE inhibitory activity and are therefore useful as a medicament for prevention and/or treatment of diseases such as septicemia, rheumatoid arthritis, osteoarthritis, infectious diseases, autoimmune diseases, malignant neoplasm, collagenosis, chronic ulcerative colitis, MOF and insulin-independent diabetes.

## Claims

1. A propenohydroxamic acid derivative represented by the following formula (1): 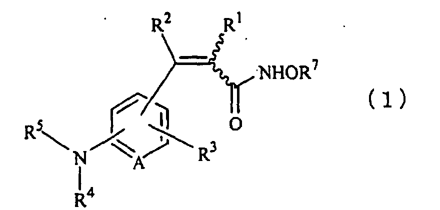 [wherein, R¹ represents a hydrogen atom, an alkyl group or a halogen atom, R² represents a cycloalkyl group, a substituted or unsubstituted aryl group or a substituted or unsubstituted heteroaryl group, R³ represents a hydrogen atom or a halogen atom, R⁴ represents a hydrogen atom, a substituted or unsubstituted alkyl group or a substituted or unsubstituted alkenyl group, R⁵ represents R⁶CO-, R⁶SO₂-, R⁶NHCO- or R⁶NHCS- (in which, R⁶ represents a substituted or unsubstituted alkyl or cycloalkyl group, a cyclic amino group, a substituted or unsubstituted aryl group or a substituted or unsubstituted heteroaryl group), R⁷ represents a hydrogen atom or a protecting group and A represents CH, a nitrogen atom or an oxidized nitrogen atom], or salt thereof.

2. A medicament comprising a propenohydroxamic acid derivative or salt thereof as claimed in Claim 1.

3. A medicament of Claim 2, which is a preventive and/or remedy for a disease selected from septicemia, rheumatoid arthritis, osteoarthritis, infectious diseases, autoimmune diseases, malignant neoplasm, collagenosis, chronic ulcerative colitis, MOF and insulin-independent diabetes.

4. A TACE inhibitor comprising a propenohydroxamic acid derivative or salt thereof as claimed in Claim 1.

5. A pharmaceutical composition comprising a propenohydroxamic acid derivative or salt thereof as claimed in Claim 1 and a pharmaceutically acceptable carrier.

6. A pharmaceutical composition of Claim 5 which is a preventive and/or remedy for a disease selected from septicemia, rheumatoid arthritis, osteoarthritis, infectious diseases, autoimmune diseases, malignant neoplasm, collagenosis, chronic ulcerative colitis, MOF and insulin-independent diabetes .

7. Use of a propenohydroxamic acid derivative or salt thereof as claimed in Claim 1 for the preparation of a medicament.

8. A method of treating a disease selected from septicemia, rheumatoid arthritis, osteoarthritis, infectious diseases, autoimmune diseases, malignant neoplasm, collagenosis, chronic ulcerative colitis, MOF and insulin-independent diabetes, which comprises administering a propenohydroxamic acid derivative or salt thereof as claimed in Claim 1.
